# EUROPEAN PATENT APPLICATION

(11) **EP 4 772 618 A1**
(43) Date of publication of application: **08.07.2026**
(21) Application number: 24858013.6
(22) Date of filing: 28.06.2024
(51) Int. Cl.: C12N 9/02, C12N 15/53, C12N 15/62, C12N 15/82, A01H 3/00, A01H 5/00, A01H 4/00

(54) **MODIFIED RNA DEMETHYLASE AND ENCODING NUCLEIC ACID THEREOF, USE THEREOF, PLANT CULTIVATION METHOD AND PRODUCT**

(30) Priority: 31.08.2023 CN 202311117642
(71) Applicant: Epiplant Co. Ltd., Beijing 100000 (CN)
(72) Inventor: LI, Ji, Beijing 100000 (CN); LI, Weifeng, Beijing 100000 (CN)
(74) Representative: Adares PartGmbB
(86) International application number: PCT/CN2024/102218
(87) International publication number: WO 2025/044469

(57) **Abstract**

Provided are a modified RNA demethylase, a nucleic acid encoding the modified RNA demethylase, the use thereof, a plant cultivation method, and a product. Compared with an original RNA demethylase derived from algae or invertebrates, the modified RNA demethylase is obtained by means of adding a specific peptide fragment sequence at the N-terminus or C-terminus of the original RNA demethylase. The modified RNA demethylase or the nucleic acid encoding the modified RNA demethylase is introduced into an initial plant to obtain a target plant having optimized traits compared with the initial plant.

## Description

### Technical Field

This invention relates to the field of genetic engineering and plant cultivation, specifically to modified RNA demethylases, nucleic acids encoding the modified RNA demethylases, use, plant cultivation methods, and products.

### Background Technology

Methods of artificial intervention in plant cultivation typically encompass various approaches, such as genetic engineering, cell engineering, and microbial engineering, among others. To obtain new plant varieties, the aforementioned methods are commonly employed. The new plant varieties here include the entire plant, plant organs (such as flowers, fruits, seeds, roots, stems, leaves, etc.), plant tissues, plant cells, and other parts that can be derived from plants.

The newly obtained plant varieties are usually expected to exhibit more optimized traits, such as optimized growth, increased yield, optimized biomass, optimized structure, or optimized cell division, among others. Among the aforementioned optimized traits, increased yield can be achieved through various means, such as an increase in the size or weight of individual seeds/thousand-grain seeds, an increase in the number of root tillers, and other factors that may affect plant yield, especially the desired increase in the yield of the parts obtained from the plant.

The aforementioned expectations are currently often achieved through certain modification methods, such as altering the DNA or histones of plants to achieve the purpose of changing plant traits. Prior to this invention, there have been studies on achieving the optimization of plant traits through the demethylation of the 6mA site in plant RNA.

### Summary of the Invention

The aforementioned methods can indeed obtain plants with optimized traits, particularly those with increased yield, as specifically referenced in the applicant's prior invention patent application, which has been granted patent in multiple countries, CN108949806A. However, in terms of trait optimization results, the introduction of original RNA demethylase or its encoding DNA derived from algae or invertebrates into plants demonstrates significantly lower trait optimization effects compared to the introduction of original RNA demethylase or its encoding DNA derived from humans or vertebrates. Furthermore, from the perspective of public acceptance, the introduction of original RNA demethylase or its encoding DNA derived from algae or invertebrates is clearly more acceptable than the introduction of original RNA demethylase or its encoding DNA derived from humans or other vertebrates. To this end, the applicant has found through research that by utilizing genetic engineering to modify the original RNA demethylase or its encoding DNA derived from algae or invertebrates, the modified RNA demethylase or its encoding DNA, after specific modifications, can achieve better trait optimization effects in plants, reaching or even exceeding the trait optimization effects of original RNA demethylase or its encoding DNA derived from humans or other vertebrates.

Accordingly, the modified RNA demethylase and its encoding nucleic acid provided by this invention, upon introduction into plants, can further optimize plant traits and make them more suitable for widespread use.

Research has found that during the growth and development of plants, specific enzymes can promote the methylation of RNA bases. For example, methyltransferase MTA can facilitate the N6-methylation of the adenine base in RNA. Here, "6-" refers to the sixth N counted from the N in the adenine base connected to the RNA sequence, either in forward or reverse order. After methylation, the RNA undergoes subsequent processes such as expression, and either the RNA itself or its resulting products can promote plant growth and development. Experiments have confirmed that removing these specific methylation enzymes can affect plant growth and development (e.g., plants may fail to produce viable seeds or seeds may not germinate properly). However, research has shown that RNA with base methylation, after partial demethylation, is more conducive to plant growth and development, thereby optimizing plant traits. Partial demethylation can be achieved using certain inducing agents, such as demethylase.

This invention study has found that introducing the modified RNA demethylase provided by this invention into plants optimizes the plants' own traits, particularly resulting in an increase in yield or biomass.

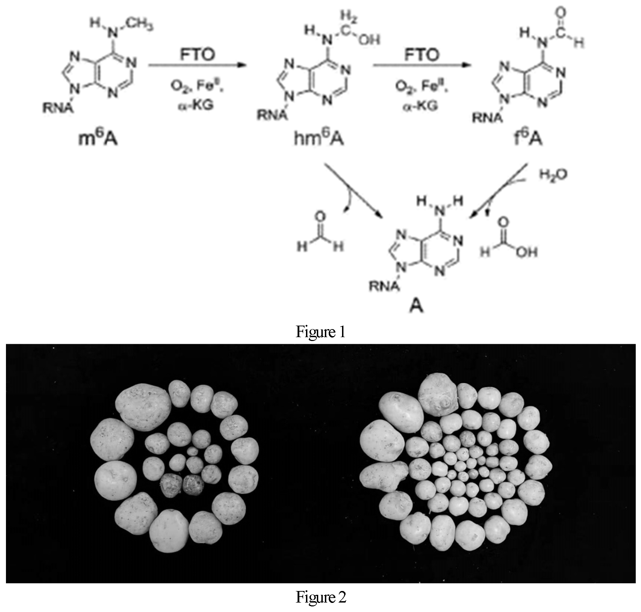

This type of RNA demethylase primarily targets RNA with already methylated bases, and its inducing chemical mechanism is illustrated in Figure 1, taking the process of demethylation of N6-methyladenine as an example. Studies has revealed that although the specific RNA sequences and the exact positon of demethylation induced by such inducer are not yet clear, it is certain that such inducer can indeed demethylate RNA with already methylated bases, thereby obtaining trait-optimized plants.

This invention provides a solution directed to the original RNA demethylase derived from algae or invertebrates, which has undergone genetic modification. By adding at least one specific peptide sequence provided in this invention to at least one end of its N-terminus or C-terminus, a modified RNA demethylase is obtained. After introducing the resulting modified RNA demethylase or its encoding nucleic acid into plants, the traits of the obtained plants exhibit optimization, particularly in terms of increased yield or biomass.

The specific peptide sequence described in this invention contains at least three amino acids selected from P, K, or R. Here, P is the abbreviation for the amino acid Pro, K is the abbreviation for the amino acid Lys, and R is the abbreviation for the amino acid Arg. The phrase "at least three amino acids selected from P, K, or R" means that the amino acids can be repeatedly or non-repeatedly selected from P, K, or R, such as combinations of P, K, K; R, R, R; or P, K, R, etc. Apart from the above three specifically selected amino acids, the other amino acids in the peptide sequence can be selected from the currently available conventional amino acids, and the conventional abbreviations for amino acids are used herein.

Preferably, in the peptide sequence described in this invention, at least two of the aforementioned three amino acids selected from P, K, or R are consecutive, which means that any two amino acids selected from P, K, or R are consecutive, while the remaining amino acid may either be consecutive with the aforementioned two amino acids or not.

Optional, the peptide sequences described in this invention can vary, such as RPRK, RKVIK, RNKKKK, PKKKRKV, KRPAATKKAGQAKKKK, TLLLRETMNNLGVSDHAVLSRKTPQPY, KRTPTAEERERGAKKLRLLEELEDTWLPYL, etc.

The term "plant" as used herein includes whole living plants, progenitors and descendants of plants, as well as parts of plants, including seeds, branches, stems, leaves, roots, flowers, and tissues and organs. The term "plant" also includes plant cells, suspension cultures, callus tissues, embryos, meristematic regions, gametophytes, sporophytes, pollen, and microspores. The term "products obtained from inactivatin treatment of the target plants" is not limited to the aforementioned parts but may also extend to harvestable parts of the plants of the present invention, such as, but not limited to, whole plants, flowers, fruits, seeds, roots, leaves, stems, etc.

Plants particularly applicable in the method of this invention include algae, ferns, and all plants belonging to the superfamily of the Plantae kingdom, especially monocotyledons and dicotyledons, including plants selected from the following usage classifications: grain crops (e.g., rice, com, soybean plant plant, etc.), forage crops (e.g., alfalfa, etc.), fiber crops (e.g., cotton, cannabis, etc.), oil crops (e.g., sesame, peanuts, etc.), sugar crops (e.g., sugarcane, beet, etc.), beverage crops, spice crops, condiment crops, medicinal crops (e.g., ginseng, Ganoderma lucidum, Fritillaria, etc.), dye crops, ornamental crops, fruit crops, vegetable crops, etc.; or specifically selected from at least one of the following plants: rapeseed plant (Brassica napus L.), tomato (Lycopersicon esculentum Mill.), lettuce (Lactuca sativa L. var. ramosa Hort.), beet (Beta vulgaris L.); or preferably selected from at least one of rice (Oryza sativa L.), com (Zea mays L.), soybean plant (Glycine max (Linn.) Merr.), potato (Solanum tuberosum), wheat (Triticum aestivum L.), millet (Setaria italica var. germanica (Mill.) Schred.), sugarcane (Saccharum officinarum), sorghum (Sorghum bicolor (L.) Moench), cassava (Manihot esculenta Crantz); or preferably selected from at least one of tobacco (Nicotiana tabacum L.), alfalfa (Medicago Sativa Linn), rubber grass (Taraxacum kok-saghyz Rodin), cotton (Gossypium spp), flax (Linum usitatissimum L.), sunflower (Helianthus annuus L.), camelina (Camelina sativa (L.) Crantz), nutsedge (Nutsedge L.), cannabis (Cannabis sativa L.), poplar (Populus L.).

The term "Initial Plant" as used herein refers to a plant that has not been processed by the cultivation method of this invention, and its structure and type may specifically include any of the aforementioned plants. The source of the "Initial Plant" may be a plant obtained from the wild or through artificial cultivation.

The term "Target Plant" as used herein refers to a plant that has been treated by the cultivation method of this invention and is expected to be obtained, the structure and type of which may likewise encompass any of the aforementioned plants.

The term "a product derived from inactivating treatment of plant " as used herein specifically refers to the product obtained after inactivatin treatment of the aforementioned "plants," which are inactive; it is not limited to any particular fields, such as industrial, medicinal, or edible uses, nor is it restricted to specific component products, such as dried granules, powder, oil, fat, fatty acids, starch, or protein, etc. Similarly, it is not confined to specific product forms, such as solid, liquid, gaseous, or mixed states, etc.

The term "algae-derived" herein refers to being derived from algae, which are a type of eukaryotic organisms within the Protista kingdom, including various types such as, but not limited to, blue algae, green algae, black algae, red algae, brown algae, and the algae used in the embodiments of this invention.

The term "increased yield" as defined herein refers to the increase in weight of a specific harvestable part relative to the initial plant. For example, in the case of rice, the increase in the weight of the rice grains is considered the increased yield, and for potatoes, the increase in the weight of the tubers is considered the increased yield, and so forth.

The term "increased biomass" as defined herein refers to the weight added to the entire plant, excluding the yield increase evaluation mentioned above; for example, in the case of rice, the increased weight of the remaining parts, excluding the rice grains, constitutes the increased biomass.

The yield and biomass mentioned herein are measured based on the dry weight of plants during the same period.

Therefore, the present invention provides a method for increasing plant yield and biomass, which includes introducing the modified RNA demethylase or its encoding nucleic acid as described in this invention into the initial plant, and obtaining a target plant with optimized traits compared to the initial plant.

The traits of plants can be adjusted and optimized by introducing the modified RNA demethylase or its encoding nucleic acid as described in this invention. Alternatively, the traits of plants can also be adjusted and optimized by using homologs, variants/conjugates, or other equivalents of the modified RNA demethylase or its encoding nucleic acid as described in this invention.

The term "modified RNA demethylase or its encoding nucleic acid" as defined herein refers to a inducer capable of inducing demethylation of RNA in plants. Specifically, it may refer to, for example, catalyzing or participating in any reaction, preferably catalyzing. Furthermore, "RNA" includes one or more types of RNA in plants, such as ribosomal RNA (rRNA), messenger RNA (mRNA), transfer RNA (tRNA), and non-coding RNA. "Demethylation" refers to the removal of methyl groups from already methylated RNA, which may occur at one or more sites on the RNA, such as the demethylation of already methylated bases in RNA, for example, the demethylation of its N6-methylated bases. Here, "6-" defines the position of methylation, which may, for example, be counted from the N starting position of the base connected to the RNA as position 1, with position 6 being the sixth position counted clockwise or counterclockwise. Here, "base" typically refers to adenine (A), among others.

The source of the "original RNA demethylase" may be any one or more sources from algae or invertebrates, such as green algae. As defined herein, "source" may refer to a direct source from algae or invertebrates, or an indirect source from algae or invertebrates, such as orthologs or paralogs derived from algae or invertebrates, preferably orthologs or paralogs derived from green algae or Lingula anatina.

The term "paralogous" involves gene duplication within the genome of a certain species, resulting in paralogous genes.

The term "orthologous" refers to homologous genes in different organisms that result from speciation.

The term "homology" refers to homologous sequences with equivalent functions to the described "original RNA demethylase".

The term "variant/conjugate" used herein refers to variants/conjugates with equivalent functions obtained through mutation, conjugation, or other methods using the "original RNA demethylase."

The term "introduction" in this text encompasses one or more aspects, such as introduction methods, introduction areas, introduction quantities, etc.

The introduction methods can be varied, for instance, by infecting plants with a vector carrying the "original RNA demethylase," inserting the "original RNA demethylase" into the plant's DNA, or by inserting a vector carrying the "original RNA demethylase" into cells, without specific limitation on the introduction area, such as the cell nucleus or cytoplasm. Furthermore, the introduction can be in small amounts or in excess, depending on the characteristics of the plant and the genes introduced.

The "nucleic acid encoding modified RNA demethylase" described herein can be obtained directly from the original nucleic acid through certain modifications to obtain nucleic acid capable of encoding modified RNA demethylase, or it can be obtained through the amino acid sequence of the modified RNA demethylase of this inveniton, using conventional reverse engineering in the field to obtain various nucleic acids capable of encoding its amino acid sequence.

The "Target Plant" described herein may be any subsequent generation of plants cultivated from the initial plant.

In the method provided by this invention, any improvement or beneficial increase in the traits of plants is considered trait optimization. Furthermore, the degree of trait optimization defined herein, denoted as Y, can also be limited by specific numerical values, which are based on a comparison with the same trait of the initial plant at the same period.

The degree of trait optimization Y can be calculated as a multiple increase compared to the initial plant, where Y is the multiple increase compared to the initial plant during the same period, Y≥3.50, preferably Y≥3.80, more preferably Y≥4.00. Here, the multiple increase can be, for example, a weight increase multiple.

The term defined herein as "DNA and/or protein, the homologs or variants/conjugates thereof with equivalent function" refers to substances that possess a certain degree of full sequence identity with DNA and/or protein. The full sequence identity is prioritized in increasing order as homologous sequences with equivalent function compared to the sequences listed in the sequence list SEQ ID NOs:1 ^{~}48, variants/conjugates. The term also indicates that it can be a DNA or protein, or a substance mixed with DNA and protein; furthermore, it refers to paralogs or orthologs with the aforementioned homology to DNA or protein, or substances obtained through corresponding vectors, hybridization, or fusion. It should be understood that "DNA and/or protein, the homologs or variants/conjugates thereof with equivalent function" is not limited to the sequences listed in the sequence list.

"DNA and/or proteins, their functional equivalents, homologs or variants/complexes" may be any natural or synthetic substances.

The term "hybridization" as defined herein refers to the process of complementary nucleotide sequences, which are essentially homologous, returning to each other. The hybridization process can occur entirely in solution, meaning that the complementary nucleic acids are both in solution. The hybridization process can also proceed in such a manner that one of the complementary nucleic acids is fixed to a substrate such as magnetic beads, agarose beads, or any other resin. Furthermore, the hybridization process can proceed in such a manner that one of the complementary nucleic acids is fixed to a solid-phase support such as a nitrocellulose membrane or nylon membrane, or fixed to a support such as silica glass through techniques such as photolithography. To enable hybridization to occur, nucleic acid molecules are typically thermally or chemically denatured to separate the double strands into two single strands and/or to remove hairpins or other secondary structures from single-stranded nucleic acids. The stringency of hybridization is influenced by conditions such as temperature, salt concentration, and the composition of the hybridization buffer.

Nucleic acid molecules or their variants may originate from any natural or artificial source. Nucleic acids/genes or their variants can be isolated from non-mammalian sources. They can be modified from their natural form in terms of composition and/or genomic environment through rigorous artificial manipulation. The nucleic acids are preferably derived from algae, particularly from sources such as green algae.

The term "homolog" encompasses two special forms of homology, namely orthologous sequences and paralogous sequences, which involve evolutionary concepts used to describe genetic relationships.

The homologous substance can also be in the form of a protein "insertion variant," where one or more amino acid residues are introduced into predetermined positions within the protein. The insertion may include fusion at the amino terminusand/or carboxyl terminus, as well as insertion within the sequence of single or multiple amino acids. Typically, insertions within the amino acid sequence will be smaller than fusions at the amino or carboxyl terminus, approximately ranging from 1 to 10 residues. Examples of fusion proteins or peptides at the amino or carboxyl terminusinclude binding domains or activation domains of transcription activators used in yeast two-hybrid systems, phage coat proteins, (histidine)6-tags, glutathione S-transferase tags, protein A, maltose-binding protein, dihydrofolate reductase, Tag.100 epitope, c-myc epitope, FLAG epitope, lacZ, CMP (calmodulin-binding peptide), HA epitope, C protein epitope, and VSV epitope.

Functional variants applicable to the methods of this invention also include optional splice variants of nucleic acid molecules or genes. The term "optional splice variants" as used herein encompasses such variants of nucleic acid sequences in which selected introns and/or exons have been removed, replaced, or added. Such variants are those in which the biological activity of the protein remains unaffected, and can be obtained by selectively retaining functional fragments of the protein. These splice variants can be naturally occurring or artificially created. Methods for producing such splice variants are well known in the field.

The "N-terminus" or "C-tenninus" mentioned in this invention specifically refers to the two ends of the original protein.

The "original protein" described in this invention specifically refers to proteins that have not been processed by the modification methods of this invention, which can be proteins of natural origin or artificially modified proteins.

In conclusion, the solutions provided by the present invention are not limited to the examples listed below; further solutions consistent with the spirit and scope of the invention can be obtained through combinations, feature transfers, or other modifications based on these examples.:
First solution provided by the present invention:
Optional, when the peptide sequence has the amino acid sequence characteristics of a), wherein at least two amino acids selected from the group consisting of H, K, and R are consecutive, or the sequence contains V or I.

Optionally, the peptide sequence has any of the following amino acid sequence characteristics:
(K/R)(K/R)X₁₋₂(K/R), (K/R)X₁₋₂(K/R)(K/R), (K/R)(K/R)X1_{0.12}(K/R)₂₋₅, (R/K/H)X₂₋₅PY, or K(V/I)XKX₁₋₂(K/H/R), wherein X represents any amino acid.

Optionally, the peptide sequence includes at least one of the following: RPRK, RKVIK, RNKKKK, PKKKRKV, KRPAATKKAGQAKKKK, TLLLRETMNNLGVSDHAVLSRKTPQPY, KRTPTAEERERGAKKLRLLEELEDTWLPYL.

Optionally, the original RNA demethylase includes at least one of its homologs, variants, or conjugates with equivalent function.

Optionally, the original RNA demethylase promotes the demethylation of RNA N6-methyladenine.

Optionally, the modified RNA demethylase is as shown in Seq.ID.No: 13 ^{~}Seq.ID.No:48.

Optionally, the invertebrates include at least one of Acanthaster planci or sacoglossan.

Optionally, the algae include at least one of blue algae, red algae, cryptophytes, dinoflagellates, chrysophytes, yellow-green algae, diatoms, brown algae, euglenoids, green algae, and charophytes
Optionally, the modified RNA demethylase promotes demethylation of N6-methyladenine in RNA.

Second solution provided by the present invention:
A nucleic acid encoding the aforementioned modified RNA demethylase.

Optionally, the nucleic acid is DNA.

Third solution provided by the present invention:
The use of the aforementioned modified RNA demethylase or nucleic acid for introduction into plants.

Optionally, the application includes any one of yield increase or biomass increase.

Optionally, the plants include at least one of rapeseed plant, tomato, lettuce, beet, rice, corn, soybean plant, potato, wheat, millet, sugarcane, sorghum, cassava, tobacco, alfalfa, rubber grass, cotton, flax, sunflower, camelina, nutsedge, cannabis, and poplar.

Fourth solution provided by the present invention:
A method for plant cultivation, wherein the modified RNA demethylase or nucleic acid is introduced into the initial plant, and a target plant with optimized traits compared to the initial plant is obtained.

Optionally, trait optimization includes at least one of yield increase or biomass increase.

Optionally, the modified RNA demethylase or nucleic acid is introduced in an amount excessive for the initial plant.

Optionally, the initial plants are selected from at least one of rapeseed, tomato, lettuce, beet, rice, com, soybean plant, potato, wheat, millet, sugarcane, sorghum, cassava, tobacco, alfalfa, rubber grass, cotton, flax, sunflower, camelina, nutsedge, cannabis, and poplar.

Optionally, the introduced plant part includes at least one of plant organs, plant tissues, or plant cells.

Optionally, the introduced plant part is selected from plant meristem.

Optionally, the plant part subject to introduction treatment includes at least one of the cell nucleus and cytoplasm (cell sap).

Optional methods of introduction include introducing a vector carrying the modified RNA demethylase or the aforementioned nucleic acid into the plant.

Optionally, compared to the initial plant, the trait optimization degree is Y, where Y represents the fold increase relative to the initial plant at the same period, Y ≥ 3.50, preferably Y ≥ 3.80, more preferably Y ≥ 4.00.

Fifth solution provided by the present invention:
The target plants obtained by the aforementioned plant cultivation method include at least one of the following: the whole living plant, plant organs, plant tissues, or plant cells.

Sixth solution provided by the present invention:
A product obtained from inactivatin treatment of the aforementioned target plant.

### Illustration of the Attached Figure

Figure 1 is a schematic diagram of the demethylation process of N6-methyladenine.
Figure 2 is a comparative diagram of the tubers of plants obtained by the experimental group and the blank group, using potatoes as an example.
Figure 3 is a comparative image of the entire plant obtained from the experimental group and the blank group, using rice as an example.

### Specific Implementation Method

In order to enable persion skilled in the art to better understand the technical solution of the present invention, the following provides a further detailed description of the present invention in conjunction with specific embodiments.

### Sequence modification of original RNA demethylase derived from algae or invertebrates.

The raw RNA demethylase obtained from various algae or invertebrates is subjected to gene sequencing, screening, and amplification to obtain the corresponding sequence samples. The sequences of the raw RNA demethylase from various algae sources are shown in the sequence list SEQ.ID.NO: 1^{~}12. Each sequence is directly subjected to PCR and recombined into the p1307-FLAG vector to obtain p1307-RNA demethylase-FLAG. Subsequently, the RNA demethylase-FLAG fragment is amplified and recombined into the pGEX-His vector to obtain pGEX-RNA demethylase-FLAG-His, which can be used for subsequent in vitro experiments and peptide sequence introduction. The plasmid pGEX-RNA demethylase-FLAG-His was circularized and recombined using primers containing the peptide sequence, and the resulting modified gene fragment was subsequently reintroduced into the p1307-FLAG vector. This results in the modified RNA demethylase of this invention, with specific sequences as shown in the sequence list SEQ.ID.NO: 13^{~}48. The obtained modified RNA demethylase can introduce peptide sequences at least at one end of the N-terminus or C-terminus of the original protein. For example, introducing a peptide sequence at the N-terminus: inserting the NLS sequence between the first amino acid M and the second amino acid of the original protein. Similarly, introducing a peptide sequence at the C-terminus: adding the NLS sequence after the last amino acid of the original protein.

### Embodiment 1: Method for Obtaining Target Plants through Agrobacterium Infection of Callus Tissue

### 1. Inducing callus tissue using plant seeds as test material.

### 1) Sterilization:

Take mature plant seeds, manually remove the shells, and select seeds that are plump, clean, and free of sterile spots. Place the seeds into a 100ml sterile beaker, pour in 70% alcohol for disinfection for 2 minutes; discard the alcohol, add 20% sodium hypochlorite (NaClO) solution, and soak for 30 minutes; discard the sodium hypochlorite solution, rinse the seeds 4-5 times with sterile distilled water, and finally soak the seeds in sterile distilled water for 30 minutes.

### 2) Induction Culture (requires aseptic operation):

After sterilizing the seeds, place them on sterile filter paper to absorb surface moisture, then transfer them into NB medium containing 2.0mg/L 2,4-D (pH 5.8), with 12-14 seeds per dish. To ensure the induction rate, it is best to maintain the germination direction of the seeds parallel or slightly downward to the medium during inoculation, avoiding upward or vertically downward directions. After the operation, seal the culture dishes with sealing film and incubate them in a light incubator at 30°C with approximately 50% humidity. Conduct dark induction culture for 20-30 days until obvious loose callus tissue appears, then proceed with subculture or preculture transformation.

### 3) Subculture (requires aseptic operation):

Open the petri dish on the ultra-clean workbench, use tweezers to select callus tissue that has naturally split, is growing vigorously, has a hard texture, a tender yellow color, and a particle diameter of approximately 3mm, and place it into NB medium containing 2.0mg/L. 2,4-D and 0.5mg/L 6-BA (pH 5.8). Place 10 pieces of callus tissue in each dish and incubate in the dark at 30°C. If the callus tissue becomes severely softened, it is recommended to transfer it to light for subculture. The subculture duration is approximately 10 to 15 days (depending on the growth of the callus tissue, determine the next subculture time), with a total of two subcultures. Note: If a significant amount of water is found on the lid during the petri dish culture process, it is unsuitable and may lead to culture failure. The reason is that the bottom of the petri dish is heated, and the lid temperature is lower than the medium temperature, causing water vapor to condense on the petri dish lid

### 2. Agrobacterium Culture

Experimental Group: Transfer the vector carrying the modified DNA or modified protein of this invention and the hygromycin resistance gene Hygromycin into the parasitic fungus, for example, transfer the pCAMBIA 1307 vector carrying the modified DNA or modified protein of this invention and the hygromycin resistance gene Hygromycin into Agrobacterium LBA4404, and inoculate it onto YEP solid medium containing 20 mg/L rifampicin (Rif) and 50 mg/L kanamycin (Kan).

Control group: The original DNA or original protein and the vector containing the hygromycin resistance gene Hygromycin are introduced into the parasitic fungus, for example, the pCAMBIA 1307 vector containing the hygromycin resistance gene Hygromycin is introduced into Agrobacterium LBA4404 and inoculated onto YEP solid medium containing 20 mg/L rifampicin (Rit) and 50 mg/L kanamycin (Kan).

Blank group: Transfer the hygromycin resistance gene Hygromycin vector into the parasitic fungus, for example, transfer the hygromycin resistance gene Hygromycin pCAMBIA 1307 vector into Agrobacterium LBA4404, and inoculate it onto YEP solid medium containing 20mg/L rifampicin (Rif) and 50mg/L kanamycin (Kan).

The control group, experimental group, and blank group were cultured at 28°C for 2 days, after which Agrobacterium monoclonal colonies were selected for colony PCR to verify whether the induction medium had been transferred into the Agrobacterium. Positive monoclonal colonies were then selected and cultured in 4 ml YEP medium (containing 50 mg/L Kan and 20 mg/L Rif) at 28°C, with shaking at 220 rpm for 20^{~}36 hours until the bacterial solution reached an OD600 of 0.8^{~}1.0.

### 3. Co-cultivation and Infection

1) Cultivate the Agrobacterium suspension at 4°C, 4000 rpm, centrifuge for 10 minutes, and remove the supernatant. Prepare a suspension using AAM medium containing 100 µmol/L acetosyringone, adjusting the final OD600 concentration of the bacterial suspension to approximately 0.2.
2) Pick out the rice callus that has grown to a certain size and place it into the Agrobacterium suspension for infection for 20-30 minutes.
3) Take out the callus and place it on sterile filter paper to drain for 20-30 minutes, so as to avoid excessive growth of Agrobacterium during co-cultivation, which may cause excessive damage to the callus.
4) Place the callus tissue in NB medium containing 2.0mg/L. 2,4-D and 100umol/L acetosyringone (pH 5.2), incubate in the dark at 25°C for 48^{∼}72 hours.

Note: Co-cultivation requires tracking and observation. If Agrobacterium becomes apparent, it indicates excessive infection, which may lead to bacterial growth during subsequent screening.

### 4. Screening of Resistant Callus

Take out the callus tissue and wash it 5^{~}6 times with sterile water, continuously shaking during the process. Then place the callus tissue on sterilized filter paper to air dry, and evenly distribute it on NB medium containing 50mg/L hygromycin (pH 5.8) for the first round of selection screening. Incubate in the dark at 28°C, and if mold or Agrobacterium growth is observed, promptly transfer the contaminated callus tissue to a new selection plate.

After approximately 30 days of screening, once new callus tissue has grown, transfer it to fresh NB medium containing 50 mg/L hygromycin (pH 5.8) for further screening for 7^{~}10 days. If significant growth is observed, it is considered positive callus tissue; if no growth occurs, even if browning or death does not appear, it may still be a false positive.

### 5. Induction, Differentiation, and Rooting of Positive Callus.

Place the second round of screened, vigorously growing, yellow callus granules (ensuring that the callus tissue used for differentiation is flawless) onto NB medium (pH 5.8) containing 2.0 mg/L 6-BA, 0.5 mg/L kinetin, and 50 mg/L hygromycin. Place 2-3 positive clones per bottle, with a small amount of callus tissue for each clone. The callus tissue placed should be of high quality, regardless of quantity. Incubate in the dark at 27°C for 10 days, followed by light-induced differentiation for 10-20 days until green leaves emerge, after which rooting can proceed. Light intensity should be 4000 lux, 14 h/d.

Place each robust seedling differentiated from the clones into 1/2N6 medium (pH 5.8) containing 0.5mg/L naphthaleneacetic acid and 50mg/L hygromycin to induce rooting, with 2-3 seedlings per bottle. Cultivate under light at 27^{∼}30°C, with a light intensity of 4000 lux for 14 h/d. After 7^{~}10 days, open the sealing film and add an appropriate amount of sterile water. Transplant after 2^{~}3 days.

Note: During the differentiation process, callus subculture is not allowed. The time from differentiation to transplantation for the target plant is approximately three months.

### 6. Acclimation and Transplantation of Target Plants

Select the seedlings of the target plant with well-differentiated roots and stems/leaves (when the seedlings grow to the top of the test tube, open the cap in a timely manner), remove the sealing film, add an appropriate amount of distilled water or sterile water (to prevent the culture medium from growing bacteria), acclimate the seedlings for about 3 days to a week, then wash off the agar, transplant them into soil pots in the greenhouse for growth, test, and obtain the target plant.

### 7. Detection and Verification of Target Plants

1) PCR Test: Design induction medium primers, for example, RNA demethylase-F: ATGAAGCGCACCCCGACTG; RNA demethylase-R: GGGTTITGCTTCCAGAAGCTGA; the PCR reaction program is: 95°C for 5 minutes, 95°C for 15 seconds, 58°C for 15 seconds, 72°C for 30 seconds, 35 cycles, 72°C extension for 10 minutes, stored at 4°C. After the reaction is completed, the PCR product is analyzed by 1% agarose gel electrophoresis.
2) Method for Rapid Detection of Target Plants with Hygromycin: Cut and collect fresh green leaves approximately 1cm in length from the seedlings to be tested (with incisions at both ends), and lay them flat on the detection medium (0.7% agar, 1ml/L 6-BA, 50mg/L hygromycin) at 28°C, with a 16h light/8h dark cycle for 48 hours. Leaves that remain fresh green indicate positive plants, while leaves of negative seedlings exhibit block necrosis.

### 8. Evaluation of the Effects of Inducer in Target Plants

The data was collected from target plants in the experimental group during the maturity stage and target plants in the control group. The measured data primarily focuses on the increase in yield of each plant, expressed in percentage (%). The weight involved in the aforementioned data refers to dry weight, specifically obtained by measuring after killing green in a 105°C oven for 20 minutes and drying in an 80°C oven for 20 hours.

### Embodiment 2: A Method for Obtaining Target Plants through Agrobacterium Infection of Shoot Apical Tissues.

### 1. Using mature plant embryos as experimental materials to induce callus tissue.

Soak mature seeds in clean water, changing the water every 1-2 hours. After 2 days, when the seeds begin to split and expose the white embryo, conduct germination and rooting at 37°C. Once the embryo grows to 1.5-2.0 cm, use a scalpel to remove the embryo and coleoptile from the stem apex and stem ring, exposing the apical meristem of the stem.

### 2. Agrobacterium Culture (same as Step 2 in Embodiment 2)

### 3. Co-cultivation and Infection

Place the processed plants into the AAM resuspension solution (see Appendix of Embodiment 1), add 2001/L of the surfactant Silwet L-77, vacuum treat at 1.5Kpa for 8 minutes, discard the resuspension solution, and place on clean, moist perlite, incubate in the dark at 28°C for 3 days.

### 4. Screening, Transplanting, and Management of Target Plants

Move the plants cultivated in the dark to the plant seedbed, and at the three to four leaf stage, collect leaves from the transformed plants for PCR detection and identification of transformed seedlings: design gene primers, such as RNA demethylase-F: ATGAAGCGCACCCCGACTG; RNA demethylase-R: GGGTTTTGCTTCCAGAAGCTGA; PCR reaction program: 95°C for 5 minutes, 95°C for 15 seconds, 58°C for 15 seconds, 72°C for 30 seconds, 35 cycles, 72°C extension for 10 minutes, preservation at 4°C. After the reaction ends, perform 1% agarose gel electrophoresis analysis on the PCR products. Transplant the surviving plants to the transgenic plant demonstration base greenhouse after 7 days, with regular fertilization and water management. Harvest the seeds of the transformed seedlings.

### 5. Evaluation of the traits of the target plant (same as Step 8 in Embodiment 2)

### Embodiment 3: A Method for Obtaining Target Plants Through Agrobacterium Infection and Induction of Meristem Formation

### 1. Plant Cultivation

Plant mature seeds in nutrient soil, and grow them to maturity (approximately 63^{~}66 days) in a greenhouse environment at 24°C with 16 hours of light and 8 hours of darkness.

### 2. Agrobacterium Culture

DRs (Growth Regulatory Elements) are a class of growth regulatory elements that induce the formation of meristem in plants, commonly used ones include BBM, ipt, ΔMP, STM, Wus2, etc. The vector carrying the induction medium, Hygromycin resistance gene, and DRs is transferred into Agrobacterium GV3101. It is cultured overnight at 28°C for 12 hours in growth medium (10mM MES, pH5.6, 20µM acetosyringone, 50mg/L kanamycin, 50mg/L gentamicin), then centrifuged at 5000rpm for 10 minutes to collect the bacteria, which are resuspended in infiltration medium (10mM MES, 150µM acetosyringone, 10mM MgCl2) to achieve an OD600 value of 0.2^{~}0.3. Before inoculation, the Agrobacterium infection solution is incubated at room temperature (approximately 25°C) for 2^{~}4 hours.

### 3. Infection and Target Plants Screening

Remove visible branch meristems from all plants, leaving 2-3 nodes and supporting leaves. Immediately use a syringe and a 31G needle to inoculate the Agrobacterium infection solution at the wound site. Observe bud formation at the cut site of the plant 38-48 days after inoculation. Each injection site with newly formed tissue or meristem is counted as a single event. Perform genomic PCR identification on the appearance of bud tissue (see Embodiment 2, Step 7) as an indicator of the presence and expression of transgenes.

### 4. Transplantation and Management of Target Plants

After cutting the newly generated bud tissue and transferring it to rooting medium to form complete seedlings, transplant the surviving plants to the greenhouse for conventional fertilization and water management. Harvest the seeds of the transformed seedlings.

### 5. Evaluation of the traits of the target plant (same as Step 8 in Example 2)

### Example 4: Method for Obtaining Target Plants through Mechanical Inoculation with PVX Vector

### 1. Plant Cultivation

Plant mature seeds in nutrient soil, and grow them to maturity (approximately 63~66 days) in a greenhouse environment at 24°C with 16 hours of light and 8 hours of darkness.

### 2. Agrobacterium Culture

Transfer the vector pPZPVX carrying the induction medium into Agrobacterium C58C1, collect the bacterial cells after culturing in Luria broth medium, and suspend them in infection buffer (10mM MES [pH5.8], 10mM MgCl2, 100µg/mL acetosyringone) to achieve an OD600 value of 0.2~0.3. Prior to inoculation, incubate the Agrobacterium infection solution at room temperature (approximately 25°C) for 2~4 hours.

### 3. Infection and Target Plants Screening

Inject Agrobacterium carrying the plasmid expressing the induction medium into tobacco leaves using a syringe. Seven days later, homogenize the inoculated leaves with 10mM NaPi buffer (pH 7.0) and verify the expression of the induction medium via western blot. After homogenization, centrifuge at 16,000g for 3 minutes and filter the supernatant using a 0.45µm filter. Apply the filtered supernatant along with carborundum (600 mesh) onto the fourth or fifth true leaf, gently tub by hand to mechanically inoculate PVX. After inoculation, place the plants under a 16-hour light/8-hour dark cycle at 16°C for growth, and observe the formation of new buds at the inoculation site 7 to 8 days later.

Note: Western blot detection of inducer expression: Mix the powdered leaf tissue after inoculation with 1× loading buffer, heat at 95°C for 10 minutes, centrifuge at 15,000 rpm at room temperature for 10 minutes, and collect the supernatant. Load onto 10% SDS-PAGE, transfer the membrane after electrophoresis, block, and use inducer antibodies to detect the expression of the inducer.

### 4. Transplantation and Management of Target Plants

After cutting the newly generated bud tissue and transferring it to rooting medium to form complete seedlings, transplant the surviving plants to the greenhouse for conventional fertilization and water management. Harvest the seeds of the transformed seedlings.

### 5. Evaluation of the traits of the target plant (same as Step 8 in Embodiment 2)

### Embodiment 5: Method for Obtaining Target Plants through Haploid Induction-Edit (HI-Edit) Technology

Haploid inducing technology (HI) can induce haploids through appropriate modification of CENTROMERIC HISTONE H3 or knockout of MATRILINEAL, followed by chromosome doubling, thereby obtaining homozygous candidate materials in a short period of time, fixing the genotype of crops, which is of significant importance in crop breeding practice. Haploid induction-edit technology (HI-Edit) combines haploid induction breeding with gene editing technology, enabling direct improvement of a wide range of plants, including commercial varieties, in a short time, freeing from the limitations of existing gene editing systems imposed by species and genotypes. Since the chromosomes of the donor carrying gene-editing elements introduced through haploid-inducing lines are eliminated during the hybridization process, the resulting hybrids do not contain genetic information derived from the patemal parent, thereby eliminating the need for multiple backcrosses. Chromosome doubling of the target haploid plants alone can yield improved diploid lines, shortening the breeding cycle. It can improve target crops through distant hybridization, has a wide range of applications, and can obtain transgene-free lines.

### 1. Construct the inducing medium vector and introduce it into the non-haploid inducible inbred line NP2222 to obtain stable transformed plants.

Using 9-day-old immature embryos isolated from the self-crossing line NP2222, the isolated immature embryos are mixed with Agrobacterium strain LBA4404 carrying the vector with induction medium. The infected embryos are cultured on recovery medium and callus induction medium. Callus is selected on medium containing mannose, and resistant callus is regenerated into target plants to obtain stably transformed plants.

### 2.Evaluation of the Traits of Target Plants (same as Step 8 in Example II)

The aforementioned Embodiments 1 to 5 can all be methods of introducing modified DNA or modified proteins into the initial plant to obtain the target plant in this invention, and the degree of trait optimization is measured for each. The yield and biomass data of the experimental group, control group, and blank group are measured respectively, using the blank group as the standard. The average increase in yield and biomass of the experimental group and control group relative to the blank group is based on dry weight. The following table data is presented using the data from Embodiment 1, and the data from the other examples differ from that of Embodiment 1 by no more than 0.05.

**Table 1-MRP 1-Mono- Ostreococcus lucimarinus**

| Plant | Yield Evaluation Section | Increase in yield relative to the experimental group | Increase in yield relative to the control group | The increase in biomass of the experimental group (excluding the yield evaluation section) | Increase in Biomass of the Control Group (Excluding Yield Evaluation Section) |
|---|---|---|---|---|---|
| Rice | Rice | 3.53 | 3.41 | 3.74 | 2.85 |
| Potato | Tuber | 3.88 | 3.37 | 3.52 | 3.19 |
| Com | Seed | 3.98 | 3.35 | 3.72 | 3.14 |
| Soybean plant | Seed | 3.85 | 3.38 | 3.71 | 2.82 |
| plant | | | | | |
| Tomato | Fruit | 3.87 | 3.43 | 3.72 | 2.84 |
| Wheat | Seed | 4.28 | 3.15 | 3.79 | 2.70 |
| Sugarcane | Stem | 3.75 | 3.26 | 3.73 | 2.83 |
| Sorghum | Seed | 3.70 | 3.40 | 3.74 | 3.21 |
| Rapeseed plant | Seed | 3.55 | 3.22 | 3.55 | 2.86 |
| Lettuce | Stem and Leaves | 4.14 | 3.39 | 3.70 | 3.17 |
| Beetroot | Stem and Leaves | 4.32 | 3.12 | 3.66 | 2.68 |
| Tobacco | Stem and Leaves | 3.56 | 3.37 | 3.73 | 3.40 |
| Alfalfa | Stem and Leaves | 4.20 | 3.37 | 3.78 | 3.20 |
| Rubber Grass | Root | 4.05 | 3.30 | 3.63 | 3.19 |
| Cotton | Seed Cotton | 4.27 | 3.32 | 3.60 | 3.32 |
| Amazon | Seed | 3.98 | 3.06 | 3.75 | 3.21 |
| Sunflower | Seed | 3.81 | 3.43 | 3.58 | 3.23 |
| Amaranth | Seed | 4.09 | 3.06 | 3.80 | 3.38 |
| Nutsedge | Stem and Leaves | 3.51 | 3.31 | 3.50 | 2.57 |
| Cannabis | Stem and Leaves | 3.83 | 3.12 | 3.65 | 2.89 |
| Poplar | Stem and Leaves | 4.31 | 3.25 | 3.56 | 2.73 |
| Cassava | Root | 4.24 | 3.32 | 3.69 | 3.14 |

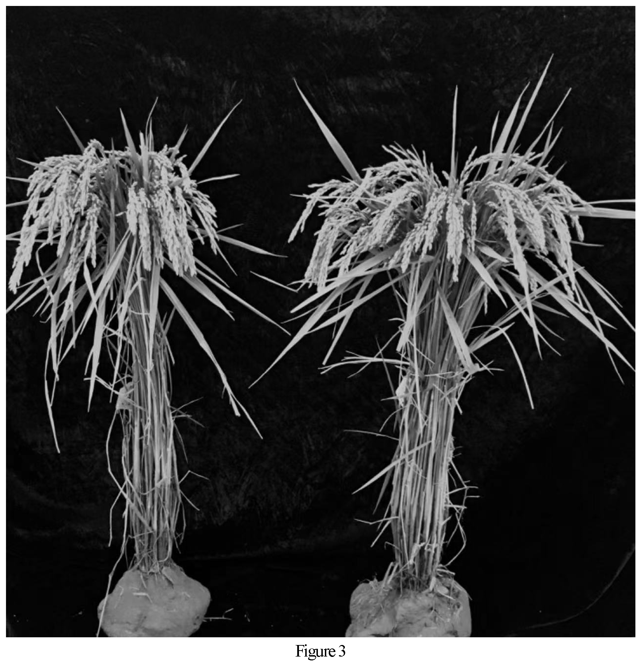

The data in the table for the control group can be calculated by comparing the average value obtained from the original RNA demethylase sequence shown in Seq.ID.NO:1 or its encoding nucleic acid with the blank group. The data for the experimental group in the table can be calculated by comparing the average value obtained from the modified RNA demethylase sequence shown in Seq.ID.NO:13^{∼}15 or its encoding nucleic acid with the blank group. Figure 2 shows the comparison between the experimental group and the blank group for potatoes in Table 1, where the left image in Figure 2 represents the blank group, and the right image represents the experimental group. Figure 3 shows the comparison between the experimental group and the blank group for rice in Table 1, where the left image in Figure 3 represents the blank group, and the right image represents the experimental group.

**Table 2-MRP_2-Mono-Aphanomyces astaci**

| Plant | Yield Evaluation | Increase in yield relative to | Increase in yield relative | The increase in biomass of the | Increase in Biomass of the |
|---|---|---|---|---|---|
| | Section | the experimental group | to the control group | experimental group (excluding the yield evaluation section) | Control Group (Excluding Yield Evaluation Section) |
| Rice | Rice | 3.77 | 3.18 | 3.78 | 3.02 |
| Potato | Tuber | 3.56 | 3.14 | 3.50 | 3.30 |
| Com | Seed | 3.98 | 3.32 | 3.59 | 2.93 |
| Soybean plant plant | Seed | 4.31 | 3.06 | 3.55 | 3.44 |
| Tomato | Fruit | 3.94 | 3.20 | 3.74 | 2.58 |
| Wheat | Seed | 3.85 | 3.31 | 3.62 | 3.28 |
| Sugarcane | Stem | 4.09 | 3.12 | 3.59 | 3.31 |
| Sorghum | Seed | 3.97 | 3.16 | 3.77 | 3.06 |
| Rapeseed plant | Seed | 4.26 | 3.36 | 3.61 | 3.33 |
| Lettuce | Stem and Leaves | 4.20 | 3.44 | 3.55 | 3.03 |
| Beetroot | Stem and Leaves | 4.10 | 3.21 | 3.66 | 3.38 |
| Tobacco | Stem and Leaves | 4.12 | 3.21 | 3.61 | 2.56 |
| Alfalfa | Stem and Leaves | 3.84 | 3.28 | 3.51 | 2.95 |
| Rubber Grass | Root | 3.86 | 3.13 | 3.77 | 2.93 |
| Cotton | Seed Cotton | 4.35 | 3.25 | 3.70 | 3.33 |
| Amazon | Seed | 3.62 | 3.13 | 3.68 | 2.79 |
| Sunflower | Seed | 4.26 | 3.26 | 3.74 | 2.57 |
| Amaranth | Seed | 3.61 | 3.34 | 3.57 | 3.08 |
| Nutsedge | Stem and Leaves | 3.70 | 3.45 | 3.60 | 2.84 |
| Cannabis | Stem and Leaves | 4.26 | 3.45 | 3.67 | 2.58 |
| Poplar | Stem and Leaves | 4.32 | 3.26 | 3.55 | 3.45 |
| Cassava | Root | 4.06 | 3.16 | 3.71 | 3.26 |

The control group data in the table can be calculated by comparing the average value derived from the original RNA demethylase sequence shown in Seq.ID.NO:2 or its encoding nucleic acid with the blank group. The experimental group data in the table can be calculated by comparing the average value derived from the modified RNA demethylase sequences shown in Seq.ID.NO: 16^{~}18 or their encoding nucleic acids with the blank group.

**Table 3-MRP 3-Mono-Micromonas commoda**

| Plant | Yield Evaluation Section | Increase in yield relative to the experimental group | Increase in yield relative to the control group | The increase in biomass of the experimental group (excluding the yield evaluation section) | Increase in Biomass of the Control Group (Excluding Yield Evaluation Section) |
|---|---|---|---|---|---|
| Rice | Rice | 3.75 | 3.21 | 3.7 | 2.64 |
| Potato | Tuber | 4.25 | 3.36 | 3.68 | 2.90 |
| Com | Seed | 4.29 | 3.11 | 3.55 | 3.01 |
| Soybean plant plant | Seed | 3.65 | 3.40 | 3.65 | 2.76 |
| Tomato | Fruit | 3.91 | 3.35 | 3.65 | 2.59 |
| Wheat | Seed | 4.07 | 3.35 | 3.57 | 3.09 |
| Sugarcane | Stem | 3.63 | 3.19 | 3.56 | 3.29 |
| Sorghum | Seed | 3.61 | 3.11 | 3.51 | 2.89 |
| Rapeseed plant | Seed | 4.13 | 3.11 | 3.54 | 2.96 |
| Lettuce | Stem and Leaves | 4.14 | 3.31 | 3.77 | 3.06 |
| Beetroot | Stem and Leaves | 4.18 | 3.39 | 3.65 | 2.96 |
| Tobacco | Stem and Leaves | 4.26 | 3.23 | 3.55 | 2.61 |
| Alfalfa | Stem and Leaves | 3.81 | 3.11 | 3.59 | 2.56 |
| Rubber Grass | Root | 3.87 | 3.37 | 3.50 | 2.99 |
| Cotton | Seed Cotton | 3.73 | 3.13 | 3.60 | 2.69 |
| Amazon | Seed | 3.51 | 3.35 | 3.75 | 2.80 |
| Sunflower | Seed | 4.26 | 3.29 | 3.55 | 2.81 |
| Amaranth | Seed | 3.89 | 3.12 | 3.66 | 3.30 |
| Nutsedge | Stem and Leaves | 3.65 | 3.16 | 3.65 | 2.84 |
| Cannabis | Stem and Leaves | 4.35 | 3.35 | 3.65 | 2.66 |
| Poplar | Stem and Leaves | 4.25 | 3.32 | 3.60 | 2.89 |
| Cassava | Root | 3.66 | 3.05 | 3.68 | 3.04 |

The data of the control group in the table can be calculated by comparing the average value obtained from the original RNA demethylase sequence or its encoding nucleic acid as shown in Seq.ID.NO:3 with the blank group. The data of the experimental group in the table can be calculated by comparing the average value obtained from the modified RNA demethylase sequence or its encoding nucleic acid as shown in Seq.ID.NO:19^{∼}21 with the blank group.

**Table 4-MRP_4-Mono-Micromonas pusilla**

| Plant | Yield Evaluation Section | Increase in yield relative to the experimental group | Increase in yield relative to the control group | The increase in biomass of the experimental group (excluding the yield evaluation section) | Increase in Biomass of the Control Group (Excluding Yield Evaluation Section) |
|---|---|---|---|---|---|
| Rice | Rice | 4.06 | 3.31 | 3.53 | 2.77 |
| Potato | Tuber | 3.65 | 3.41 | 3.59 | 3.45 |
| Com | Seed | 4.21 | 3.29 | 3.68 | 2.72 |
| Soybean plant plant | Seed | 3.81 | 3.29 | 3.54 | 2.83 |
| Tomato | Fruit | 4.08 | 3.15 | 3.80 | 3.30 |
| Wheat | Seed | 4.21 | 3.19 | 3.62 | 3.44 |
| Sugarcane | Stem | 4.33 | 3.25 | 3.68 | 2.60 |
| Sorghum | Seed | 4.11 | 3.25 | 3.72 | 2.91 |
| Rapeseed plant | Seed | 3.99 | 3.11 | 3.51 | 2.74 |
| Lettuce | Stem and Leaves | 3.67 | 3.13 | 3.66 | 3.17 |
| Beetroot | Stem and Leaves | 4.13 | 3.42 | 3.60 | 3.32 |
| Tobacco | Stem and Leaves | 3.66 | 3.27 | 3.57 | 3.07 |
| Alfalfa | Stem and Leaves | 3.79 | 3.33 | 3.58 | 2.60 |
| Rubber Grass | Root | 4.08 | 3.31 | 3.71 | 3.41 |
| Cotton | Seed Cotton | 4.07 | 3.11 | 3.50 | 3.01 |
| Amazon | Seed | 4.12 | 3.3 | 3.52 | 2.65 |
| Sunflower | Seed | 4.07 | 3.22 | 3.65 | 3.35 |
| Amaranth | Seed | 4.17 | 3.06 | 3.78 | 3.32 |
| Nutsedge | Stem and Leaves | 3.72 | 3.14 | 3.63 | 3.06 |
| Cannabis | Stem and Leaves | 4.00 | 3.07 | 3.56 | 3.05 |
| Poplar | Stem and Leaves | 3.66 | 3.22 | 3.74 | 3.31 |
| Cassava | Root | 3.87 | 3.32 | 3.53 | 3.06 |

The data of the control group in the above table can be calculated by comparing the original RNA demethylase sequence shown in Seq.ID.NO:4 or its encoding nucleic acid with the blank group to obtain the average value. The data of the experimental group in the above table can be calculated by comparing the modified RNA demethylase sequence shown in Seq.ID.NO:22~24 or its encoding nucleic acid with the blank group to obtain the average value.

**Table 5-MRP_5-Mono-Polarella glacialis**

| Plant | Yield Evaluation Section | Increase in yield relative to the experimental group | Increase in yield relative to the control group | The increase in biomass of the experimental group (excluding the yield evaluation section) | Increase in Biomass of the Control Group (Excluding Yield Evaluation Section) |
|---|---|---|---|---|---|
| Rice | Rice | 3.82 | 3.08 | 3.66 | 2.92 |
| Potato | Tuber | 3.95 | 3.25 | 3.66 | 3.11 |
| Com | Seed | 3.68 | 3.41 | 3.54 | 2.90 |
| Soybean plant plant | Seed | 3.78 | 3.33 | 3.79 | 2.97 |
| Tomato | Fruit | 4.34 | 3.20 | 3.61 | 2.96 |
| Wheat | Seed | 4.15 | 3.15 | 3.78 | 2.79 |
| Sugarcane | Stem | 3.93 | 3.08 | 3.72 | 2.69 |
| Sorghum | Seed | 4.03 | 3.23 | 3.55 | 2.60 |
| Rapeseed plant | Seed | 3.99 | 3.24 | 3.61 | 2.71 |
| Lettuce | Stem and Leaves | 3.83 | 3.24 | 3.58 | 2.61 |
| Beetroot | Stem and Leaves | 4.18 | 3.17 | 3.67 | 3.28 |
| Tobacco | Stem and Leaves | 4.34 | 3.34 | 3.61 | 3.13 |
| Alfalfa | Stem and Leaves | 4.31 | 3.42 | 3.64 | 3.28 |
| Rubber Grass | Root | 4.17 | 3.41 | 3.72 | 2.73 |
| Cotton | Seed Cotton | 4.29 | 3.36 | 3.71 | 2.98 |
| Amazon | Seed | 4.25 | 3.33 | 3.71 | 3.22 |
| Sunflower | Seed | 3.61 | 3.4 | 3.66 | 3.05 |
| Amaranth | Seed | 3.51 | 3.19 | 3.56 | 2.85 |
| Nutsedge | Stem and Leaves | 4.11 | 3.10 | 3.79 | 2.70 |
| Cannabis | Stem and Leaves | 3.59 | 3.29 | 3.68 | 2.79 |
| Poplar | Stem and Leaves | 3.61 | 3.11 | 3.72 | 2.89 |
| Cassava | Root | 3.67 | 3.27 | 3.79 | 2.65 |

The data of the control group in the table can be calculated by comparing the original RNA demethylase sequence shown in Seq.ID.NO:5 or its encoding nucleic acid with the blank group to obtain the average value. The data of the experimental group in the table can be calculated by comparing the modified RNA demethylase sequences shown in Seq.ID.NO:25^{∼}27 or their encoding nucleic acids with the blank group to obtain the average value.

**Table 6-MRP 6-Mono-Guillardia theta**

| Plant | Yield Evaluation Section | Increase in yield relative to the experimental group | Increase in yield relative to the control group | The increase in biomass of the experimental group (excluding the yield evaluation section) | Increase in Biomass of the Control Group (Excluding Yield Evaluation Section) |
|---|---|---|---|---|---|
| Rice | Rice | 3.77 | 3.44 | 3.80 | 2.57 |
| Potato | Tuber | 3.66 | 3.19 | 3.78 | 2.93 |
| Com | Seed | 3.50 | 3.40 | 3.62 | 2.58 |
| Soybean plant plant | Seed | 3.99 | 3.23 | 3.55 | 3.08 |
| Tomato | Fruit | 4.00 | 3.07 | 3.74 | 3.39 |
| Wheat | Seed | 4.01 | 3.45 | 3.78 | 2.63 |
| Sugarcane | Stem | 3.53 | 3.28 | 3.72 | 3.10 |
| Sorghum | Seed | 4.15 | 3.38 | 3.58 | 2.86 |
| Rapeseed plant | Seed | 3.84 | 3.45 | 3.56 | 3.41 |
| Lettuce | Stem and Leaves | 3.92 | 3.39 | 3.54 | 3.29 |
| Beetroot | Stem and Leaves | 4.32 | 3.35 | 3.53 | 2.62 |
| Tobacco | Stem and Leaves | 4.13 | 3.23 | 3.75 | 3.28 |
| Alfalfa | Stem and Leaves | 3.87 | 3.13 | 3.55 | 2.88 |
| Rubber Grass | Root | 4.27 | 3.26 | 3.71 | 3.07 |
| Cotton | Seed Cotton | 4.27 | 3.43 | 3.77 | 3.13 |
| Amazon | Seed | 3.62 | 3.08 | 3.62 | 2.99 |
| Sunflower | Seed | 4.09 | 3.12 | 3.65 | 3.28 |
| Amaranth | Seed | 3.87 | 3.19 | 3.60 | 2.81 |
| Nutsedge | Stem and Leaves | 4.03 | 3.34 | 3.67 | 2.83 |
| Cannabis | Stem and Leaves | 3.65 | 3.45 | 3.58 | 2.76 |
| Poplar | Stem and Leaves | 3.73 | 3.14 | 3.64 | 2.91 |
| Cassava | Root | 4.11 | 3.30 | 3.56 | 2.80 |

The data of the control group in the table can be calculated by comparing the average value obtained from the original RNA demethylase sequence or its encoding nucleic acid as shown in Seq.ID.NO:6 with the blank group. The data of the experimental group in the table can be calculated by comparing the average value obtained from the modified RNA demethylase sequence or its encoding nucleic acid as shown in Seq.ID.NO:28^{∼}30 with the blank group.

**Table 7-MRP 7-Mono-Ostreococcus tauri**

| Plant | Yield | Increase in | Increase in | The increase in | Increase in |
|---|---|---|---|---|---|
| | Evaluation Section | yield relative to the experimental group | yield relative to the control group | biomass of the experimental group (excluding the yield evaluation section) | Biomass of the Control Group (Excluding Yield Evaluation Section) |
| Rice | Rice | 3.87 | 3.34 | 3.75 | 2.58 |
| Potato | Tuber | 4.18 | 3.09 | 3.63 | 3.40 |
| Com | Seed | 4.05 | 3.44 | 3.65 | 2.70 |
| Soybean plant plant | Seed | 3.73 | 3.18 | 3.62 | 2.72 |
| Tomato | Fruit | 4.25 | 3.09 | 3.63 | 3.14 |
| Wheat | Seed | 3.96 | 3.21 | 3.56 | 2.84 |
| Sugarcane | Stem | 4.08 | 3.29 | 3.66 | 3.37 |
| Sorghum | Seed | 3.96 | 3.18 | 3.64 | 3.15 |
| Rapeseed plant | Seed | 3.57 | 3.38 | 3.61 | 3.46 |
| Lettuce | Stem and Leaves | 4.16 | 3.15 | 3.63 | 2.77 |
| Beetroot | Stem and Leaves | 4.21 | 3.25 | 3.73 | 3.01 |
| Tobacco | Stem and Leaves | 3.50 | 3.14 | 3.69 | 2.60 |
| Alfalfa | Stem and Leaves | 3.91 | 3.05 | 3.54 | 3.27 |
| Rubber Grass | Root | 3.53 | 3.30 | 3.73 | 3.17 |
| Cotton | Seed Cotton | 4.16 | 3.41 | 3.76 | 2.57 |
| Amazon | Seed | 3.97 | 3.45 | 3.51 | 2.62 |
| Sunflower | Seed | 3.76 | 3.37 | 3.58 | 3.23 |
| Amaranth | Seed | 3.72 | 3.16 | 3.87 | 2.76 |
| Nutsedge | Stem and Leaves | 3.79 | 3.33 | 3.51 | 3.07 |
| Cannabis | Stem and Leaves | 3.98 | 3.09 | 3.69 | 3.21 |
| Poplar | Stem and Leaves | 4.15 | 3.07 | 3.62 | 3.45 |
| Cassava | Root | 3.54 | 3.13 | 3.66 | 3.13 |

The data of the control group in the table can be calculated by comparing the original RNA demethylase sequence shown in Seq.ID.NO:7 or its encoding nucleic acid with the blank group to obtain the average value. The data of the experimental group in the table can be calculated by comparing the modified RNA demethylase sequence shown in Seq.ID.NO:31^{∼}33 or its encoding nucleic acid with the blank group to obtain the average value.

**Table 8-MRP_11-mono- Ectocarpus sp.**

| Plant | Yield Evaluation Section | Increase in yield relative to the experimental group | Increase in yield relative to the control group | The increase in biomass of the experimental group (excluding the yield evaluation section) | Increase in Biomass of the Control Group (Excluding Yield Evaluation Section) |
|---|---|---|---|---|---|
| Rice | Rice | 4.26 | 3.07 | 3.58 | 2.56 |
| Potato | Tuber | 3.53 | 3.26 | 3.52 | 3.01 |
| Com | Seed | 3.90 | 3.38 | 3.74 | 3.16 |
| Soybean plant plant | Seed | 3.92 | 3.31 | 3.54 | 2.87 |
| Tomato | Fruit | 4.13 | 3.05 | 3.64 | 3.15 |
| Wheat | Seed | 4.11 | 3.28 | 3.69 | 3.06 |
| Sugarcane | Stem | 3.99 | 3.44 | 3.79 | 3.05 |
| Sorghum | Seed | 3.86 | 3.23 | 3.71 | 2.74 |
| Rapeseed plant | Seed | 3.88 | 3.45 | 3.73 | 3.34 |
| Lettuce | Stem and Leaves | 3.62 | 3.14 | 3.66 | 3.18 |
| Beetroot | Stem and Leaves | 4.18 | 3.44 | 3.72 | 2.99 |
| Tobacco | Stem and Leaves | 4.32 | 3.31 | 3.60 | 2.66 |
| Alfalfa | Stem and Leaves | 3.66 | 3.23 | 3.57 | 2.92 |
| Rubber Grass | Root | 3.65 | 3.19 | 3.53 | 3.23 |
| Cotton | Seed Cotton | 4.23 | 3.18 | 3.74 | 2.99 |
| Amazon | Seed | 4.17 | 3.44 | 3.64 | 2.82 |
| Sunflower | Seed | 4.02 | 3.17 | 3.69 | 2.91 |
| Amaranth | Seed | 4.31 | 3.28 | 3.67 | 3.27 |
| Nutsedge | Stem and Leaves | 3.58 | 3.32 | 3.75 | 2.81 |
| Cannabis | Stem and Leaves | 4.09 | 3.07 | 3.64 | 3.34 |
| Poplar | Stem and Leaves | 3.94 | 3.31 | 3.71 | 2.85 |
| Cassava | Root | 3.51 | 3.37 | 3.76 | 3.31 |

The data of the control group in the table can be calculated by comparing the original RNA demethylase sequence shown in Seq.ID.NO:8 or its encoding nucleic acid with the blank group to obtain the average value. The data of the experimental group in the table can be calculated by comparing the modified RNA demethylase sequence shown in Seq.ID.NO:34^{∼}36 or its encoding nucleic acid with the blank group to obtain the average value.

**Table 9-MRP 12-mono- Conferva siliculosa**

| Plant | Yield Evaluation Section | Increase in yield relative to the experimental group | Increase in yield relative to the control group | The increase in biomass of the experimental group (excluding the yield evaluation section) | Increase in Biomass of the Control Group (Excluding Yield Evaluation Section) |
|---|---|---|---|---|---|
| Rice | Rice | 3.91 | 3.32 | 3.67 | 2.86 |
| Potato | Tuber | 3.77 | 3.06 | 3.73 | 3.06 |
| Com | Seed | 4.16 | 3.12 | 3.58 | 2.66 |
| Soybean plant plant | Seed | 3.82 | 3.08 | 3.55 | 3.43 |
| Tomato | Fruit | 3.66 | 3.25 | 3.57 | 2.85 |
| Wheat | Seed | 3.98 | 3.34 | 3.55 | 3.15 |
| Sugarcane | Stem | 4.02 | 3.26 | 3.76 | 2.58 |
| Sorghum | Seed | 3.51 | 3.14 | 3.52 | 3.26 |
| Rapeseed plant | Seed | 4.31 | 3.26 | 3.63 | 2.77 |
| Lettuce | Stem and Leaves | 3.77 | 3.27 | 3.79 | 2.76 |
| Beetroot | Stem and Leaves | 4.33 | 3.15 | 3.65 | 2.69 |
| Tobacco | Stem and Leaves | 4.38 | 3.18 | 3.68 | 2.61 |
| Alfalfa | Stem and Leaves | 4.12 | 3.14 | 3.53 | 3.20 |
| Rubber Grass | Root | 3.56 | 3.23 | 3.60 | 3.36 |
| Cotton | Seed Cotton | 3.52 | 3.44 | 3.51 | 2.71 |
| Amazon | Seed | 4.31 | 3.31 | 3.80 | 3.07 |
| Sunflower | Seed | 4.30 | 3.37 | 3.74 | 3.01 |
| Amaranth | Seed | 3.86 | 3.41 | 3.77 | 2.88 |
| Nutsedge | Stem and Leaves | 3.80 | 3.22 | 3.70 | 2.65 |
| Cannabis | Stem and Leaves | 4.20 | 3.41 | 3.73 | 2.63 |
| Poplar | Stem and Leaves | 4.03 | 3.33 | 3.62 | 2.61 |
| Cassava | Root | 4.17 | 3.15 | 3.59 | 2.63 |

The data of the control group in the table can be calculated by comparing the average value obtained from the original RNA demethylase sequence or its encoding nucleic acid as shown in Seq.ID.NO:9 with the blank group. The data of the experimental group in the table can be calculated by comparing the average value obtained from the modified RNA demethylase sequence or its encoding nucleic acid as shown in Seq.ID.NO:37^{∼}39 with the blank group.

**Table 10-Fragilariopsis cylindrus**

| Plant | Yield Evaluation Section | Increase in yield relative to the experimental group | Increase in yield relative to the control group | The increase in biomass of the experimental group (excluding the yield evaluation section) | Increase in Biomass of the Control Group (Excluding Yield Evaluation Section) |
|---|---|---|---|---|---|
| Rice | Rice | 3.84 | 3.13 | 3.51 | 2.80 |
| Potato | Tuber | 4.34 | 3.05 | 3.51 | 3.28 |
| Com | Seed | 3.65 | 3.37 | 3.58 | 3.41 |
| Soybean plant plant | Seed | 4.12 | 3.36 | 3.69 | 2.64 |
| Tomato | Fruit | 3.54 | 3.45 | 3.57 | 2.84 |
| Wheat | Seed | 3.60 | 3.43 | 3.74 | 2.85 |
| Sugarcane | Stem | 4.07 | 3.36 | 3.70 | 3.22 |
| Sorghum | Seed | 3.70 | 3.33 | 3.51 | 2.73 |
| Rapeseed plant | Seed | 4.20 | 3.33 | 3.63 | 2.87 |
| Lettuce | Stem and Leaves | 3.82 | 3.38 | 3.71 | 3.17 |
| Beetroot | Stem and Leaves | 3.73 | 3.21 | 3.75 | 2.80 |
| Tobacco | Stem and Leaves | 3.86 | 3.38 | 3.65 | 2.88 |
| Alfalfa | Stem and Leaves | 4.06 | 3.41 | 3.70 | 2.73 |
| Rubber Grass | Root | 3.85 | 3.27 | 3.50 | 2.69 |
| Cotton | Seed Cotton | 3.96 | 3.33 | 3.52 | 3.38 |
| Amazon | Seed | 4.08 | 3.27 | 3.79 | 2.99 |
| Sunflower | Seed | 3.59 | 3.25 | 3.55 | 3.07 |
| Amaranth | Seed | 3.72 | 3.06 | 3.75 | 3.40 |
| Nutsedge | Stem and Leaves | 4.25 | 3.34 | 3.77 | 3.21 |
| Cannabis | Stem and Leaves | 4.35 | 3.45 | 3.67 | 3.32 |
| Poplar | Stem and Leaves | 4.02 | 3.17 | 3.63 | 3.25 |
| Cassava | Root | 4.04 | 3.37 | 3.81 | 3.30 |

The data of the control group in the above table can be calculated by comparing the original RNA demethylase sequence shown in Seq.ID.NO: 10 or its encoding nucleic acid with the blank group to obtain the average value. The data of the experimental group in the above table can be calculated by comparing the modified RNA demethylase sequences shown in Seq.ID.NO:40^{∼}42 or their encoding nucleic acids with the blank group to obtain the average value.

**Table 11 - Crown-of- Thorns Starfish (Acanthaster planci)**

| Plant | Yield Evaluation Section | Increase in yield relative to the experimental group | Increase in yield relative to the control group | The increase in biomass of the experimental group (excluding the yield evaluation section) | Increase in Biomass of the Control Group (Excluding Yield Evaluation Section) |
|---|---|---|---|---|---|
| Rice | Rice | 4.28 | 3.18 | 3.78 | 3.29 |
| Potato | Tuber | 4.08 | 3.31 | 3.79 | 2.86 |
| Com | Seed | 4.09 | 3.13 | 3.58 | 2.92 |
| Soybean plant plant | Seed | 3.54 | 3.24 | 3.67 | 3.19 |
| Tomato | Fruit | 3.51 | 3.08 | 3.58 | 3.02 |
| Wheat | Seed | 4.14 | 3.05 | 3.77 | 2.92 |
| Sugarcane | Stem | 4.09 | 3.23 | 3.53 | 3.07 |
| Sorghum | Seed | 4.29 | 3.08 | 3.61 | 2.76 |
| Rapeseed plant | Seed | 4.23 | 3.07 | 3.80 | 3.31 |
| Lettuce | Stem and Leaves | 3.93 | 3.43 | 3.74 | 3.01 |
| Beetroot | Stem and Leaves | 3.58 | 3.35 | 3.52 | 3.43 |
| Tobacco | Stem and Leaves | 3.78 | 3.11 | 3.73 | 2.80 |
| Alfalfa | Stem and Leaves | 4.24 | 3.38 | 3.75 | 3.29 |
| Rubber Grass | Root | 4.05 | 3.43 | 3.78 | 2.88 |
| Cotton | Seed Cotton | 3.86 | 3.05 | 3.60 | 2.69 |
| Amazon | Seed | 3.91 | 3.22 | 3.62 | 2.81 |
| Sunflower | Seed | 3.79 | 3.30 | 3.74 | 2.69 |
| Amaranth | Seed | 3.51 | 3.45 | 3.75 | 3.15 |
| Nutsedge | Stem and Leaves | 3.74 | 3.40 | 3.57 | 2.87 |
| Cannabis | Stem and Leaves | 3.62 | 3.29 | 3.79 | 3.15 |
| Poplar | Stem and Leaves | 4.32 | 3.30 | 3.65 | 2.80 |
| Cassava | Root | 3.97 | 3.23 | 3.76 | 2.69 |

The data of the control group in the above table can be calculated by comparing the original RNA demethylase sequence shown in Seq.ID.NO:11 or its encoding nucleic acid with the blank group to obtain the average value. The data of the experimental group in the above table can be calculated by comparing the modified RNA demethylase sequence shown in Seq.ID.NO:43^{∼}45 or its encoding nucleic acid with the blank group to obtain the average value.

**Table 12 - Saccoglossus kowalevskii**

| Plant | Yield Evaluation Section | Increase in yield relative to the experimental group | Increase in yield relative to the control group | The increase in biomass of the experimental group (excluding the yield evaluation section) | Increase in Biomass of the Control Group (Excluding Yield Evaluation Section) |
|---|---|---|---|---|---|
| Rice | Rice | 3.68 | 3.46 | 3.63 | 3.25 |
| Potato | Tuber | 4.11 | 3.05 | 3.77 | 2.77 |
| Com | Seed | 3.95 | 3.28 | 3.56 | 3.21 |
| Soybean plant plant | Seed | 4.03 | 3.05 | 3.58 | 3.32 |
| Tomato | Fruit | 4.05 | 3.31 | 3.78 | 3.26 |
| Wheat | Seed | 3.99 | 3.32 | 3.65 | 2.95 |
| Sugarcane | Stem | 4.32 | 3.07 | 3.78 | 2.81 |
| Sorghum | Seed | 3.54 | 3.36 | 3.79 | 2.62 |
| Rapeseed plant | Seed | 3.83 | 3.18 | 3.57 | 3.29 |
| Lettuce | Stem and Leaves | 4.24 | 3.15 | 3.52 | 3.12 |
| Beetroot | Stem and Leaves | 4.23 | 3.20 | 3.77 | 3.42 |
| Tobacco | Stem and Leaves | 4.01 | 3.14 | 3.71 | 3.09 |
| Alfalfa | Stem and Leaves | 3.61 | 3.13 | 3.62 | 2.77 |
| Rubber Grass | Root | 4.18 | 3.10 | 3.77 | 2.80 |
| Cotton | Seed Cotton | 4.28 | 3.13 | 3.74 | 3.06 |
| Amazon | Seed | 4.15 | 3.34 | 3.69 | 3.13 |
| Sunflower | Seed | 3.57 | 3.33 | 3.68 | 2.68 |
| Amaranth | Seed | 3.75 | 3.14 | 3.52 | 2.81 |
| Nutsedge | Stem and Leaves | 4.13 | 3.13 | 3.71 | 2.87 |
| Cannabis | Stem and Leaves | 4.28 | 3.19 | 3.59 | 3.02 |
| Poplar | Stem and Leaves | 4.14 | 3.28 | 3.65 | 2.81 |
| Cassava | Root | 4.03 | 3.27 | 3.54 | 2.91 |

The data of the control group in the table can be calculated by comparing the original RNA demethylase sequence shown in Seq.ID.NO: 12 or its encoding nucleic acid with the blank group to obtain the average value. The data of the experimental group in the table can be calculated by comparing the modified RNA demethylase sequences shown in Seq.ID.NO:46^{∼}48 or their encoding nucleic acids with the blank group to obtain the average value.

From the above data, it can be concluded that the modified RNA demethylase derived from algae or invertebrates and its encoding nucleic acid, when introduced into plants, can result in new plants with increased yield and biomass. Furthermore, compared to the introduction of the original RNA demethylase and its encoding nucleic acid, it demonstrates a more advantageous effect.

The above is merely a preferred embodiment of the present invention. It should be noted that the aforementioned preferred embodiment should not be considered as a limitation on the present invention. The scope of protection of the present invention shall be defined by the scope of the claims. For person skilled in the art, several improvements and modifications can be made without departing from the spirit and scope of the present invention, and these improvements and modifications should also be regarded as within the scope of protection of the present invention.

### Sequence List

Sequence list information:
   DTD Version: V1_3
   Filename: 2.xml
   Software Name: WIPO Sequence
   Software Version: 2.3.0
   Date of Generation: 2023-08-30
Basic Information:
   Current Application / Intellectual Property Office: CN
   Current Application / Applicant File Name: 24060CNCNP2-1
   Applicant's Name or Title: 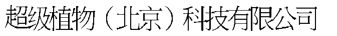
   Applicant's Name or Title / Language: zh
   Applicant's Name or Title / Latin Name: EpiPlant CO. LTD.
   Title of Invention: Modified RNA Demethylase and Its Encoding Nucleic Acid, Applications, and Methods for Plant Cultivation and Products
   Total Sequence: 48
Sequence:
   Serial Number (ID): 1
   Length: 419
   Molecular Type: AA
   Feature Location/Qualifier:
      - source, 1..419
         > mol_type, protein
         > organism, Ostreococcus lucimarinus
Residue: 
MSPSSSVLEP EDGEPFARVH RAHYRGFVVD APSVLPASLH DDVERAFDDM RSRGEFTHDV 60
VSAGNKVSTT YVRRCLLGED GMTYHYQKLR LFAQPWRGRE AYEVVRRLNE TLTRSARERC 120
EKLGGAFAES ECEYNVTLIN YMETEGESEI ELRNEEKFDL GTTSVSWHSD SSLRENSTVA 180
VYHTYEAPER KDWRVALRAL NAECEVLCVP LEDKATYYMC GEFNATHHHA VLTGSSARYS 240
STHRVAVVAK DTFQYIKRRC IDALAIVPDL ERENKPLDAK QIQFLADVHR EVEFQWIRMF 300
IILQGEAHAAW IIDTYWTRKIA ELTEAWDRME ACFRVILSKL KRSSRSPESP PRAYAMLLYA 360
LKTVKELRDE YTKRTKASAY ASLPPSQRPV DLPAYDNTSP LPFELKPVIY FLEEEQNKV 419
Serial Number (ID): 2
   Length: 522
   Molecular Type: AA
   Feature Location/Qualifier:
      - source, 1..522
         > mol_type, protein
         > organism, Aphanomyces astaci
Residue: MDPKERKKLE RQKKKAKALE RKSAKKPNVA SLKPVSQPTT SHKRPSEPSG SSAAPPAKKQ 60 
QIEKAKPLPV PSNYTGPEKF LTPSHPDFHD ILTRSYVGFV HEPAAAQPRE FHAGMRAAFE 120
HLRDKGYFQY DVVMAGGKHL SRTFVRRTLV GNFGITYKYL GLRIFAHAWD VPSSAAVFRN 180
LYALNQAIVA RTKASASPAP PSCEYNLTLI NYMEPDNPVL GLKDDLTYDM GKVSVSWHAD 240
SSLEDFSSIG VYHTLLPTKK SKKGPCDWRI AMRLSPDVPG AKTTPPLVVP TGDGDVYYLN 300
GDFNHHHQHM VLSGTAVRVS STHRVAVTAE DTLQYIQAKV TAALKSSAKD PQDKCRRGHA 360
DDVRHEQTTV TTLEMDWLRQ YWIQGDKHND LHVDWQQPML ALEASWRKLE ARTKTLADAL 420
VQSHDVDDLK VLKAVLEGLR NRQVGRDQFV HRMKDKVFKR IAADFQPMER PVWTDWDDVH 480
LLPKDLTATI AALHAHKLVL ESEKKRQWKI HAGTRHNKIN KA 522
Serial Number (ID): 3
   Length: 520
   Molecular Type: AA
   Feature Location/Qualifier:
      - source, 1..520
         > mol_type, protein
         > organism, Micromonas commode
Residue: 
MGKKHKPRPD DRARVVDASD VGGKVKKKKA NGATMKASRR KGGGASANGD GRPYDPSRTL 60
SKPAPPPPAY TPRVPTGAYI TPEHDEYDAC VARSYVGFVV DPPDILPDEL HADVAAAFKT 120
MAKRGLFTRD VLAAGKTVSP TFVSRTLVGE RGMTYHYQKL RIFALPWSDE DTPEGSPLRV 180
VRRLNDAMKR RSRALLTRHR DARVPGDVTG SCEFNVTLIN LMEPERKESV ALKDEGQFGM 240
GKASVSWHSD SSLQDTSTVA VYHQTSGGID GDDTSWHVAL KCLDGVTPAL RVPLRSHSAY 300
YIMRDFNLHH HHAVLNGDTR RFSSTHRVAV TAKDTFDYVR GRCERALAVL PSLKWRAGRW 360
PKGTKPPRDA SSTQLSDAVR VLGETHREVE FQWIRMFWLQ GTRHAAQHDG YWTRRIEELT 420
QAWDVMELGL RFAMDACARA ADGKAKAEPR AFEMLRYLLE TIAELREEHD ARTQSGAYAQ 480
VPDDCKPVDL PGFDDSSPLP RNLRPVLNTL ERHRRRALDR 520
Serial Number (ID): 4
   Length: 541
   Molecular Type: AA
   Feature Location/Qualifier:
      - source, 1..541
         > mol_type, protein
         > organism, Micromonas pusilla
Residue: 
MATATGSSGG KLEKRKKKKK KTTAVERAGS GGGGNAKKPT TTTASHRPTK PLLRAAPPPP 60
SYRLRGTSGS DDDDDDAAPS FLTPSHAEYE RCMKTSYLGF HVDDPSALPE KTHREIARAF 120
ERMASDGIFH RDVLAAGKSV SPTFVSRTLL GERGMTYHYQ RLRIFAHPWD EETQPDASHP 180
 
FRAVKRLNDA LVSRSTAVLR ASSDPRLGDG VDGSCDFNVT LINLMEPASR CAEVPLKDEA 240
LFGMGKARST SVSWHSDSSL QDVSTVAVYH QTDDDDDDSW AVALKTLDGV TPAMRTPLRS 300
REMYYMLRDF NAHHHHAVIT GESRRFSSTH RVAVVAKDTF EHIKTRCERA LRLLPGIKKI 360
AQVGPGPGDD LAAFARSAQM MGETHRECEF QWIRMFWLQG SRHAASHSAY WRQRIAELTQ 420
AWDAMELGYR WALDALRAGA STGAMPLRAY DVVSYLLEET RELRAEHARR SRAGAYEAMP 480
ADCRPVDEPK FDEKSPLPFD LGPVLATLAG WRRDAERAQT TTTEGGSKTK KGKPGKGSKR 540
R 541
Serial Number (ID): 5
   Length: 506
   Molecular Type: AA
   Feature Location/Qualifier:
      - source, 1..506
         > mol_type, protein
         > organism, Polarella glacialis
Residue: 
MDMDRFVREP ADASSESFHE EFRRCLHGLY EQGIFQYDLI QPVGPGTPVV STRVTRCLIG 60
EPGITYKYLG LRMFAHPWKG EKTSESCSAM YQHNQELISK AEEQAVAVGC DAPPYSSCFN 120
LTLINRMDPA DSGAPNKLGA VSVSWHADSS LVDYSTITVY VAEHDATQDS FGLSPARPCK 180
PCDDPWRVAL RHTPNVQGPE KSCPWEVAAQ ETAKPVKRRA KAKVHTVPIH DGDIYHLIGD 240
FNHHNQHAVL AGRDAVLAVR RGMPVLGGSG TIRYSSTHRV ALTEGHSYRS VKQRCSTALS 300
VALADMIPAA AQVHEEQQCS LELEFEWIRQ FFIQGKAHRE ALPWWHSKMD ELVFLWRQFE 360
VLTAQRLTLL RRAAAGKVGT SELDPFCLAL KNGLQKRAEL REAWRQREHD PLLKKQPANR 420
QPLQCLFDAP SDAPHGAGSE ALPHNVWPLV EEISQIQLQL HETKKVGKPW YQFVLNVYQG 480
CAFQGGEVTC LSWTLFSQRL PMKRRW 506
Serial Number (ID): 6
   Length: 543
   Molecular Type: AA
   Feature Location/Qualifier:
      - source, 1..543
         > mol_type, protein
         > organism, Guillardia theta
Residue: 
MQETLWQRVK EGLEWFDGAI GEGEEEEEAG RIAGNKGGGR RRGFEQALER YYPKFRVIHR 60
DLLPSALEPS TRRALEVLKD QMGWFRRDYV WVKGKVTQTM VTRLLVGERG CTYKYKGLRL 120
FCRSWETEAA KVPDSACHAL AEIRRLNEVL KEKSRKQHEC FMARGEGSGE GAVEGGVNSF 180
NATLINYFDE SIANHVETRP EPYYGMGRCC ISWHADDGVV EQSAIAVYNV IERAEKGEEE 240
ELGEVWKVGL KVKWEVDTPG VAVPMVNGDA YVMLSGFNEK NQHCVVCGRG SRFSSTHRVC 300
 
PQEDMYHNFY CRCRETFLQL HFPWNARGVL QEGEERRACL SKIVASLDAL EDLHDQLEFE 360
WLRQFHFQYF DLPPSELDDY WVEVIHVLAD LWQRMEIVVW IWISCCRRKS FMVGGKELRD 420
RLERLRQLTS QQKGQGQEVQ SSLPFLMKKL LRSEGAGGGR EAAGEKAIPE DALELVKATL 480
ARRLQKRQEH RIWWEENFRK RLSMGRGGEQ REGGKRKRKE GRADGLGFSL LPANLNSLVS 540
TLK 543
Serial Number (ID): 7
   Length: 420
   Molecular Type: AA
   Feature Location/Qualifier:
      - source, 1.420
         > mol_type, protein
         > organism, Ostreococcus tauri
Residue: 
MPDVLGPGDG DAYERVRSFH YDGFVVDDPE ILPEALHDDV ARAFDSLLRE GEFTHDVLSA 60
GNKVSRTFVT RTLLGDRGTT YHYQKLRLFA QPWDGEKRWE YATARRLNEA LTRRTREILR 120
GTRHGESECE YNVTLINRMD TREESEVALK DEALFDLGTT SVSWHSDSSL RENSTVAVYS 180
TVDDPERADW SVALRALGKQ CPALRVPLKD KATYYMCGDF NATHHHAVLT GSSARYSSTH 240
RVGVVEKDTF QYIKRRCLEA LALIPDLENK SASLDAKKIQ LLAEIHREVE FQWIRMFHLQ 300
GDTHARQHKT YWTQRIAELT EAWDRMEACF RAILSKLKLS PESTPQPSRA YSMMLYAMKE 360
VKELRDEHVK RISASAYAAL PESQRPVDLP QYDDTSPLPF ELKPVIYFVE AEQAKLKGES 420
Serial Number (ID): 8
   Length: 787
   Molecular Type: AA
   Feature Location/Qualifier:
      - source, 1..787
         > mol_type, protein
         > organism, Ectocarpus sp
Residue: 
MTMTTATTVA TTSRLLSPPT SEATTPIQHS NSGMEVSTEK TSHKKKRKSK RKRQQQERDT 60
SSIPDASRQS RGSSSSSTRP PPPPPPPPPT TTTTTTSKKH SRIKEKPARF VHPHLDAELR 120
RLGRSWLSPG DPLFKPALDR SYRGVRHEPG GSLPQAVHRG FRQSFDSLHE AGLFLYDTVQ 180
AGGKRLSRTF VTRTLVGDAG ITYKYLGLRL FAHPWGLGES DFRKGPGDSL ADALNDPCLA 240
DLFSNDGSGF DDNDRVADTL RKVGDLSEWM RLRAGAMLKE EVGEAGGERG SCAFNLTLIN 300
RMECSGAKRD LKPDPLFSMG KFSVSWHADS CLQDFSTIGV YHCTDPVPAE PDWGIALRVS 360
TEARVGGGGG GSGDGDGYGV SNVEKTRRKE AERGVKPAGP KAIASVAASA AGAVPEKQGE 420
QVLTPPLLIP LQSGDAYFLL DDFNHFHEHA VVSGSSTLRY SSTHRVADTD GNTFSHVQKR 480
 
CERALAGGGS AAAGGDYHIL NLAQVRAEQA LLTELESEWL NQFWVQGRRH ASLHPWWRGP 540
MALVLATWRE LERRTALTVR RLMSLRGQGG GEGVGPGGGG TAAETAGAEP AAIAKAFETV 600
LRALEVRRES RSRWGARFAD SAYRSLAADL APVVPALWPA EGSSGGDVGE EGSPLLLPQS 660
LGGIVSRLRE CYTVVYRAAS GGKKAGSAKA IAAGAASNWE LFRAASSSSS SGGGGAGKKH 720
GSALGKQQQQ QQQQQQQQRK MRGLAGQDGL EKTKKRAGGV VKGGGDGGGG RTLKGQGSRR 780
KDRVRER 787
Serial Number (ID): 9
   Length: 715
   Molecular Type: AA
   Feature Location/Qualifier:
      - source, 1..715
         > mol_type, protein
         > organism, Conferva siliculosa
Residue: 
MTTTSATTAA RSRLPSPPTS AATTPIQHSN NGTEASPEKT SNKKKAKNKR KRQQECSIPA 60
ASQQSRGSSS SSTRPPPPTT TTLKKHSRIK EKPARFVHPH LDAELRRLGR SWLSPGDPLF 120
TPALDRSYRG VRHEPAGSLP QAVHSGFRQS FDSLHEAGLF LYDTVQAGGK RLSRTFVTRT 180
LVGDAGITYK YLGLRLFAHP WGLGESDFRK GPGDGLADAL NDPCLADLFS KDGASSVRKS 240
KDNLRGSRFD DNDRVADTLR KVGDLSEWMR LRAGAMLKEE VREAGGERGS CAFNLTLINR 300
MECSGAKRDL KPDPLFSMGK CSVSWHADSC LQDFSTIGVY HCTDPVPAEP DWGIALRVST 360
EARVGGDGGG DGDGNGVSND EKTRRKEAER GLKPAGPKAV ASVAASAAEA VPEKQGEQVL 420
TPPLLIPLQS GDAYFLLDDF NHFHEHAVVS GSSTLRYSST HRVADTDGNT FSHVQKRCEQ 480
ALAGGGSAAA GGDDQILNLA QVRAEQALLT ELESEWLNQF WVQGRRHASL HPWWRGPMAL 540
VLATWRELER RTSLTVQHLM SLRGQDGGEA LWPAEGSGGG DVGEEGSTLL LPQSLGGVIS 600
RLRECYTVVY RAASGGKKAG SAKAIAAGAA SNWELFRAAS SSASSGGGGA GKKHGSALGK 660
QQQQQQQRKK RGAAGQDGLE KKKRAGGVVK GGGDGGGGRT SKGQRSRRKG RVRER 715
Serial Number (ID): 10
   Length: 417
   Molecular Type: AA
   Feature Location/Qualifier:
      - source, 1.417
         > mol_type, protein
         > organism, Fragilariopsis cylindrus
Residue: 
MLTPEDGVYF QKHLQDHYKG FVHLPVDRLE PRDFHEEFKN SLERLRDAGY YQYDVVMAGG 60
KYSSRTFVKR TLVGNPGITY KYLGLRLFAH AWSGPGCTPL MKSIGDMNQS MIKMTEQFPE 120
 
NERCDYNLTL INYMEPTTHT KVGFKDEANY GMGKVSVSWH ADSSLVDNSS IGVYHCLPTQ 180
RNSKWDWKIA LRPLAIESKD NTGSTKRDNK STTPKPVVVN TKDGDAYFLL GNFNMKHQHC 240
VLAGSQANRI SSTHRVAVTI EDTYDYILKR VKIARKRFRL QMETIPPSLQ SPVVGLDAKV 300
IRYCQRILTE VEMEWIAQYW LQGDQHDKMR VWWQKPMKTL EAYWCALEVY TYRLFEFLLS 360
RIASNDTVPI DVVKVLIIEF KTRQSFREQW DERRSDKIYQ RRVSEEFRPV ARPIFEN 417
Serial Number (ID): 11
   Length: 528
   Molecular Type: AA
   Feature Location/Qualifier:
      - source, 1..528
         > mol_type, protein
         > organism, Acanthaster planci
Residue: 
MRFLSLNVLR RWMSKMSKRR QTEDLGKEVK KKKLLSEVAR GRLQFLMPDH PDFKSLMRKS 60
YFNFAHEPST GIPVSLHEEL TWALDILRYH GYFYRDWVKV KGQTVQTPIS RTLIGEPGTT 120
YKYLGLRLFT IPWSFGHHKP ESEVELASET IGKLNEHYKA VSRRLLRKKT AEQTHLKSSP 180
SQEVAPCSSK DRPRERICVE EPKQMTHGLS IEEGTNFNVV LINYMDPEDP DLRLKPEPYY 240
GMGPLAVSWH MDGNLVRGST VAVYNHTCSS DGGDSTTDSD WMVGFKVSWD IETPGVAIPL 300
QTGESYFMLG DLNDTHQHCV VAGSQARFST THRVADCQQG TLSYIQQRCL EALGNLSQLE 360
NGEYELKSFQ AGHLDFTETV HNEVEFEWLR QFWMQGSRHA AERGGWVPAM ECLEADWNKM 420
EHMTKLAVEE ANRLGPEEGS VIAEALLPHL KERRKLRQEC LDKFPRRLLE TIHRDFLPIH 480
RPKWDNDDPS QPLPFDLKGI IRKMRLLCNG KRIKSPSDSE SSDTGLHS 528
Serial Number (ID): 12
   Length: 485
   Molecular Type: AA
   Feature Location/Qualifier:
      - source, 1.485
         > mol_type, protein
         > organism, Saccoglossus kowalevskii
Residue: 
MNPRMKRKIP RIPLVTSEGE VAGQLALPIR KQKLLSAVKE TQVKFLSPVD TDYKALVAEN 60
YDNFVVQPGT ELSSELHTRL QQGFETLLTN GYLYQHRIRF RNKTVMTPVS RTLIGEPGMT 120
YRYLNLRLFA LPWTFDDKKK LSDVEKACNA FYELNTYFKD HVNKLITEKF ASSTGRKIDE 180
TDDSLKTIIQ RNTEFKATIL NYMDPSEIRL KQEPYYNMGD IAVSWHKDES LVDGSTVAVY 240
NHVPMEARQD CSKWKVGLKV AWDITTPAVC TPLNNKDCYY MLDTMNDTHQ HCVIAGTLPR 300
YSSTHRVVES SDSTLCYIQQ RCQTALDNIG YKDQIPYLKS LETDVLQLIE EIHSEIEFEW 360
 
LRQFWMQGTQ HSQERQYWLK PMEQLQEDWK TMELMTKLMI DIVVKKKDQS HMIAKILLPS 420
LVERNELRNE WMNRYSEKEF YHIPEEHRPI YEPCWTDANS EMPLPFDLSQ HIDIIQECMD 480
TNDSQ 485
Serial Number (ID): 13
   Length: 423
   Molecular Type: AA
   Feature Location/Qualifier:
      - source, 1.423
         > mol_type, protein
         > organism, Ostreococcus lucimarinus
Residue: 
MRPRKSPSSS VLEPEDGEPF ARVHRAHYRG FVVDAPSVLP ASLHDDVERA FDDMRSRGEF 60
THDVVSAGNK VSTTYVRRCL LGEDGMTYHY QKLRLFAQPW RGREAYEVVR RLNETLTRSA 120
RERCEKLGGA FAESECEYNV TLINYMETEG ESEIELRNEE KFDLGTTSVS WHSDSSLREN 180
STVAVYHTYE APERKDWRVA LRALNAECEV LCVPLEDKAT YYMCGEFNAT HHHAVLTGSS 240
ARYSSTHRVA VVAKDTFQYI KRRCIDALAI VPDLERENKP LDAKQIQFLA DVHREVEFQW 300
IRMFHLQGEA HAAWHDTYWT RKIAELTEAW DRMEACFRVI LSKLKRSSRS PESPPRAYAM 360
LLYALKTVKE LRDEYTKRTK ASAYASLPPS QRPVDLPAYD NTSPLPFELK PVIYFLEEEQ 420
NKV 423
Serial Number (ID): 14
   Length: 426
   Molecular Type: AA
   Feature Location/Qualifier:
      - source, 1.426
         > mol_type, protein
         > organism, Ostreococcus lucimarinus
Residue: 
MSPSSSVLEP EDGEPFARVH RAHYRGFVVD APSVLPASLH DDVERAFDDM RSRGEFTHDV 60
VSAGNKVSTT YVRRCLLGED GMTYHYQKLR LFAQPWRGRE AYEVVRRLNE TLTRSARERC 120
EKLGGAFAES ECEYNVTLIN YMETEGESEI ELRNEEKFDL GTTSVSWHSD SSLRENSTVA 180
VYHTYEAPER KDWRVALRAL NAECEVLCVP LEDKATYYMC GEFNATHHHA VLTGSSARYS 240
STHRVAVVAK DTFQYIKRRC IDALAIVPDL ERENKPLDAK QIQFLADVHR EVEFQWIRMF 300
HLQGEAHAAW HDTYWTRKIA ELTEAWDRME ACFRVILSKL KRSSRSPESP PRAYAMLLYA 360
LKTVKELRDE YTKRTKASAY ASLPPSQRPV DLPAYDNTSP LPFELKPVIY FLEEEQNKVP 420
KKKRKV 426
Serial Number (ID): 15
   Length: 479
   Molecular Type: AA
   Feature Location/Qualifier:
      - source, 1.479
         > mol_type, protein
         > organism, Ostreococcus lucimarinus
Residue: 
MKRTPTAEER ERGAKKLRLL EELEDTWLPY LSPSSSVLEP EDGEPFARVH RAHYRGFVVD 60
APSVLPASLH DDVERAFDDM RSRGEFTHDV VSAGNKVSTT YVRRCLLGED GMTYHYQKLR 120
LFAQPWRGRE AYEVVRRLNE TLTRSARERC EKLGGAFAES ECEYNVTLIN YMETEGESEI 180
ELRNEEKFDL GTTSVSWHSD SSLRENSTVA VYHTYEAPER KDWRVALRAL NAECEVLCVP 240
LEDKATYYMC GEFNATHHHA VLTGSSARYS STHRVAVVAK DTFQYIKRRC IDALAIVPDL 300
ERENKPLDAK QIQFLADVHR EVEFQWIRMF HLQGEAHAAW HDTYWTRKIA ELTEAWDRME 360
ACFRVILSKL KRSSRSPESP PRAYAMLLYA LKTVKELRDE YTKRTKASAY ASLPPSQRPV 420
DLPAYDNTSP LPFELKPVIY FLEEEQNKVK RTPTAEERER GAKKLRLLEE LEDTWLPYL 479
Serial Number (ID): 16
   Length: 527
   Molecular Type: AA
   Feature Location/Qualifier:
      - source, 1..527
         > mol_type, protein
         > organism, Aphanomyces astaci
Residue: 
MRKVIKDPKE RKKLERQKKK AKALERKSAK KPNVASLKPV SQPTTSHKRP SEPSGSSAAP 60
PAKKQQIEKA KPLPVPSNYT GPEKFLTPSH PDFHDILTRS YVGFVHEPAA AQPREFHAGM 120
RAAFEHLRDK GYFQYDVVMA GGKHLSRTFV RRTLVGNFGI TYKYLGLRIF AHAWDIPSSA 180
AVFRNLYALN QAIVARTKAS ASPAPPSCEY NLTLINYMEP DNPVLGLKDD PTYDMGKVSV 240
SWHADSSLED FSSIGVYHTL LPTKKSKKGP CDWRIAMRLS PDVPGAKTTP PLVVPTGDGD 300
VYYLNGDFNH HHQHMVLSGT AVRVSSTHRV AVTAEDTLQY IQAKVTASLK SSAKDPQDKC 360
RRGHADDVRH EQTTVTTLEM DWLRQYWIQG DKHNDLHVDW QQPMLALEAS WRKLEARTKT 420
LADALVQSHD VDDLKVLKAV LEGLRNRQVG RDQFVHRMKD KVFKRIAADF QPMERPVWTD 480
WDDVHLLPKD LTATIAALHA HKLVLESEKK RQWKIHAGTR HHKNNKA 527
Serial Number (ID): 17
   Length: 538
   Molecular Type: AA
   Feature Location/Qualifier:
      - source, 1..538
         > mol_type, protein
         > organism, Aphanomyces astaci
Residue: 
MDPKERKKLE RQKKKAKALE RKSAKKPNVA SLKPVSQPTT SHKRPSEPSG SSAAPPAKKQ 60
QIEKAKPLPV PSNYTGPEKF LTPSHPDFHD ILTRSYVGFV HEPAAAQPRE FHAGMRAAFE 120
HLRDKGYFQY DVVMAGGKHL SRTFVRRTLV GNFGITYKYL GLRIFAHAWD IPSSAAVFRN 180
LYALNQAIVA RTKASASPAP PSCEYNLTLI NYMEPDNPVL GLKDDPTYDM GKVSVSWHAD 240
SSLEDFSSIG VYHTLLPTKK SKKGPCDWRI AMRLSPDVPG AKTTPPLVVP TGDGDVYYLN 300
GDFNHHHQHM VLSGTAVRVS STHRVAVTAE DTLQYIQAKV TASLKSSAKD PQDKCRRGHA 360
DDVRHEQTTV TTLEMDWLRQ YWIQGDKHND LHVDWQQPML ALEASWRKLE ARTKTLADAL 420
VQSHDVDDLK VLKAVLEGLR NRQVGRDQFV HRMKDKVFKR IAADFQPMER PVWTDWDDVH 480
LLPKDLTATI AALHAHKLVL ESEKKRQWKI HAGTRHHKNN KAKRPAATKK AGQAKKKK 538
Serial Number (ID): 18
   Length: 582
   Molecular Type: AA
   Feature Location/Qualifier:
      - source, 1..582
         > mol_type, protein
         > organism, Aphanomyces astaci
Residue: 
MKRTPTAEER ERGAKKLRLL EELEDTWLPY LDPKERKKLE RQKKKAKALE RKSAKKPNVA 60
SLKPVSQPTT SHKRPSEPSG SSAAPPAKKQ QIEKAKPLPV PSNYTGPEKF LTPSHPDFHD 120
ILTRSYVGFV HEPAAAQPRE FHAGMRAAFE HLRDKGYFQY DVVMAGGKHL SRTFVRRTLV 180
GNFGITYKYL GLRIFAHAWD IPSSAAVFRN LYALNQAIVA RTKASASPAP PSCEYNLTLI 240
NYMEPDNPVL GLKDDPTYDM GKVSVSWHAD SSLEDFSSIG VYHTLLPTKK SKKGPCDWRI 300
AMRLSPDVPG AKTTPPLVVP TGDGDVYYLN GDFNHHHQHM VLSGTAVRVS STHRVAVTAE 360
DTLQYIQAKV TASLKSSAKD PQDKCRRGHA DDVRHEQTTV TTLEMDWLRQ YWIQGDKHND 420
LHVDWQQPML ALEASWRKLE ARTKTLADAL VQSHDVDDLK VLKAVLEGLR NRQVGRDQFV 480
HRMKDKVFKR IAADFQPMER PVWTDWDDVH LLPKDLTATI AALHAHKLVL ESEKKRQWKI 540
HAGTRHHKNN KAKRTPTAEE RERGAKKLRL LEELEDTWLP YL 582
Serial Number (ID): 19
   Length: 526
   Molecular Type: AA
   Feature Location/Qualifier:
      - source, 1..526
         > mol_type, protein
         > organism, Micromonas commode
Residue: 
MRNKKKKGKK HKPRPDDRAR VVDASDVGGK VKKKKANGAT MKASRRKGGG ASANGDGRPY 60
DPSRTLSKPA PPPPAYTPRV PTGAYITPEH DEYDACVARS YVGFVVDPPD ILPDELHADV 120
AAAFKTMAKR GLFTRDVLAA GKTVSPTFVS RTLVGERGMT YHYQKLRIFA LPWSDEDTPE 180
GSPLRVVRRL NDAMKRRSRA LLTRHRDARV PGDVTGSCEF NVTLINLMEP ERKESVALKD 240
EGQFGMGKAS VSWHSDSSLQ DTSTVAVYHQ TSGGIDGDDT SWHVALKCLD GVTPALRVPL 300
RSHSAYYIMR DFNLHHHHAV LNGDTRRFSS THRVAVTAKD TFDYVRGRCE RALAVLPSLK 360
WRAGRWPKGT KPPRDASSTQ LSDAVRVLGE THREVEFQWI RMFWLQGTRH AAQHDGYWTR 420
RIEELTQAWD VMELGLRFAM DACARAADGK AKAEPRAFEM LRYLLETIAE LREEHDARTQ 480
SGAYAQVPDD CKPVDLPGFD DSSPLPRNLR PVLNTLERHR RRALDR 526
Serial Number (ID): 20
   Length: 547
   Molecular Type: AA
   Feature Location/Qualifier:
      - source, 1..547
         > mol_type, protein
         > organism, Micromonas commode
Residue: 
MGKKHKPRPD DRARVVDASD VGGKVKKKKA NGATMKASRR KGGGASANGD GRPYDPSRTL 60
SKPAPPPPAY TPRVPTGAYI TPEHDEYDAC VARSYVGFVV DPPDILPDEL HADVAAAFKT 120
MAKRGLFTRD VLAAGKTVSP TFVSRTLVGE RGMTYHYQKL RIFALPWSDE DTPEGSPLRV 180
VRRLNDAMKR RSRALLTRHR DARVPGDVTG SCEFNVTLIN LMEPERKESV ALKDEGQFGM 240
GKASVSWHSD SSLQDTSTVA VYHQTSGGID GDDTSWHVAL KCLDGVTPAL RVPLRSHSAY 300
YIMRDFNLHH HHAVLNGDTR RFSSTHRVAV TAKDTFDYVR GRCERALAVL PSLKWRAGRW 360
PKGTKPPRDA SSTQLSDAVR VLGETHREVE FQWIRMFWLQ GTRHAAQHDG YWTRRIEELT 420
QAWDVMELGL RFAMDACARA ADGKAKAEPR AFEMLRYLLE TIAELREEHD ARTQSGAYAQ 480
VPDDCKPVDL PGFDDSSPLP RNLRPVLNTL ERHRRRALDR TLLLRETMNN LGVSDHAVLS 540
RKTPQPY 547
Serial Number (ID): 21
   Length: 580
   Molecular Type: AA
   Feature Location/Qualifier:
      - source, 1..580
         > mol_type, protein
         > organism, Micromonas commode
Residue: 
MKRTPTAEER ERGAKKLRLL EELEDTWLPY LGKKHKPRPD DRARVVDASD VGGKVKKKKA 60
NGATMKASRR KGGGASANGD GRPYDPSRTL SKPAPPPPAY TPRVPTGAYI TPEHDEYDAC 120
VARSYVGFVV DPPDILPDEL HADVAAAFKT MAKRGLFTRD VLAAGKTVSP TFVSRTLVGE 180
RGMTYHYQKL RIFALPWSDE DTPEGSPLRV VRRLNDAMKR RSRALLTRHR DARVPGDVTG 240
SCEFNVTLIN LMEPERKESV ALKDEGQFGM GKASVSWHSD SSLQDTSTVA VYHQTSGGID 300
GDDTSWHVAL KCLDGVTPAL RVPLRSHSAY YIMRDFNLHH HHAVLNGDTR RFSSTHRVAV 360
TAKDTFDYVR GRCERALAVL PSLKWRAGRW PKGTKPPRDA SSTQLSDAVR VLGETHREVE 420
FQWIRMFWLQ GTRHAAQHDG YWTRRIEELT QAWDVMELGL RFAMDACARA ADGKAKAEPR 480
AFEMLRYLLE TIAELREEHD ARTQSGAYAQ VPDDCKPVDL PGFDDSSPLP RNLRPVLNTL 540
ERHRRRALDR KRTPTAEERE RGAKKLRLLE ELEDTWLPYL 580
Serial Number (ID): 22
   Length: 545
   Molecular Type: AA
   Feature Location/Qualifier:
      - source, 1..545
         > mol_type, protein
         > organism, Micromonas pusilla
Residue: 
MRPRKATATG SSGGKLEKRK KKKKKTTAVE RAGSGGGGNA KKPTTTTASH RPTKPLLRAA 60
PPPPSYRLRG TSGSDDDDDD AAPSFLTPSH AEYERCMKTS YLGFHVDDPS ALPEKTHREI 120
ARAFERMASD GIFHRDVLAA GKSVSPTFVS RTLLGERGMT YHYQRLRIFA HPWDEETQPD 180
ASHPFRAVKR LNDALVSRST AVLRASSDPR LGDGVDGSCD FNVTLINLME PASRCAEVPL 240
KDEALFGMGK ARSTSVSWHS DSSLQDVSTV AVYHQTDDDD DDSWAVALKT LDGVTPAMRT 300
PLRSREMYYM LRDFNAHHHH AVITGESRRF SSTHRVAVVA KDTFEHIKTR CERALRLLPG 360
IKKIAQVGPG PGDDLAAFAR SAQMMGETHR ECEFQWIRMF WLQGSRHAAS HSAYWRQRIA 420
ELTQAWDAME LGYRWALDAL RAGASTGAMP LRAYDVVSYL LEETRELRAE HARRSRAGAY 480
EAMPADCRPV DEPKFDEKSP LPFDLGPVLA TLAGWRRDAE RAQTTTTEGG SKTKKGKPGK 540
GSKRR 545
Serial Number (ID): 23
   Length: 557
   Molecular Type: AA
   Feature Location/Qualifier:
      - source, 1..557
         > mol_type, protein
         > organism, Micromonas pusilla
Residue: 
MATATGSSGG KLEKRKKKKK KTTAVERAGS GGGGNAKKPT TTTASHRPTK PLLRAAPPPP 60
SYRLRGTSGS DDDDDDAAPS FLTPSHAEYE RCMKTSYLGF HVDDPSALPE KTHREIARAF 120
ERMASDGIFH RDVLAAGKSV SPTFVSRTLL GERGMTYHYQ RLRIFAHPWD EETQPDASHP 180
FRAVKRLNDA LVSRSTAVLR ASSDPRLGDG VDGSCDFNVT LINLMEPASR CAEVPLKDEA 240
LFGMGKARST SVSWHSDSSL QDVSTVAVYH QTDDDDDDSW AVALKTLDGV TPAMRTPLRS 300
REMYYMLRDF NAHHHHAVIT GESRRFSSTH RVAVVAKDTF EHIKTRCERA LRLLPGIKKI 360
AQVGPGPGDD LAAFARSAQM MGETHRECEF QWIRMFWLQG SRHAASHSAY WRQRIAELTQ 420
AWDAMELGYR WALDALRAGA STGAMPLRAY DVVSYLLEET RELRAEHARR SRAGAYEAMP 480
ADCRPVDEPK FDEKSPLPFD LGPVLATLAG WRRDAERAQT TTTEGGSKTK KGKPGKGSKR 540
RKRPAATKKA GQAKKKK 557
Serial Number (ID): 24
   Length: 555
   Molecular Type: AA
   Feature Location/Qualifier:
      - source, 1..555
         > mol_type, protein
         > organism, Micromonas pusilla
Residue: 
MPKKKRKVAT ATGSSGGKLE KRKKKKKKTT AVERAGSGGG GNAKKPTTTT ASHRPTKPLL 60
RAAPPPPSYR LRGTSGSDDD DDDAAPSFLT PSHAEYERCM KTSYLGFHVD DPSALPEKTH 120
REIARAFERM ASDGIFHRDV LAAGKSVSPT FVSRTLLGER GMTYHYQRLR IFAHPWDEET 180
QPDASHPFRA VKRLNDALVS RSTAVLRASS DPRLGDGVDG SCDFNVTLIN LMEPASRCAE 240
VPLKDEALFG MGKARSTSVS WHSDSSLQDV STVAVYHQTD DDDDDSWAVA LKTLDGVTPA 300
MRTPLRSREM YYMLRDFNAH HHHAVITGES RRFSSTHRVA VVAKDTFEHI KTRCERALRL 360
LPGIKKIAQV GPGPGDDLAA FARSAQMMGE THRECEFQWI RMFWLQGSRH AASHSAYWRQ 420
RIAELTQAWD AMELGYRWAL DALRAGASTG AMPLRAYDVV SYLLEETREL RAEHARRSRA 480
GAYEAMPADC RPVDEPKFDE KSPLPFDLGP VLATLAGWRR DAERAQTTTT EGGSKTKKGK 540
PGKGSKRRPK KKRKV 555
Serial Number (ID): 25
   Length: 512
   Molecular Type: AA
   Feature Location/Qualifier:
      - source, 1..512
         > mol_type, protein
         > organism, Polarella glacialis
Residue: 
MRNKKKKDMD RFVREPADAS SESFHEEFRR CLHGLYEQGI FQYDLIQPVG PGTPVVSTRV 60
TRCLIGEPGI TYKYLGLRMF AHPWKGEKTS ESCSAMYQHN QELISKAEEQ AVAVGCDAPP 120
YSSCFNLTLI NRMDPADSGA PNKLGAVSVS WHADSSLVDY STITVYVAEH DATQDSFGLS 180
PARPCKPCDD PWRVALRHTP NVQGPEKSCP WEVAAQETAK PVKRRAKAKV HTVPIHDGDI 240
YHLIGDFNHH NQHAVLAGRD AVLAVRRGMP VLGGSGTIRY SSTHRVALTE GHSYRSVKQR 300
CSTALSVALA DMIPAAAQVH EEQQCSLELE FEWIRQFFIQ GKAHREALPW WHSKMDELVF 360
LWRQFEVLTA QRLTLLRRAA AGKVGTSELD PFCLALKNGL QKRAELREAW RQREHDPLLK 420
KQPANRQPLQ CLFDAPSDAP HGAGSEALPH NVWPLVEEIS QIQLQLHETK KVGKPWYQFV 480
LNVYQGCAFQ GGEVTCLSWT LFSQRLPMKR RW 512
Serial Number (ID): 26
   Length: 533
   Molecular Type: AA
   Feature Location/Qualifier:
      - source, 1..533
         > mol_type, protein
         > organism, Polarella glacialis
Residue: 
MDMDRFVREP ADASSESFHE EFRRCLHGLY EQGIFQYDLI QPVGPGTPVV STRVTRCLIG 60
EPGITYKYLG LRMFAHPWKG EKTSESCSAM YQHNQELISK AEEQAVAVGC DAPPYSSCFN 120
LTLINRMDPA DSGAPNKLGA VSVSWHADSS LVDYSTITVY VAEHDATQDS FGLSPARPCK 180
PCDDPWRVAL RHTPNVQGPE KSCPWEVAAQ ETAKPVKRRA KAKVHTVPIH DGDIYHLIGD 240
FNHHNQHAVL AGRDAVLAVR RGMPVLGGSG TIRYSSTHRV ALTEGHSYRS VKQRCSTALS 300
VALADMIPAA AQVHEEQQCS LELEFEWIRQ FFIQGKAHRE ALPWWHSKMD ELVFLWRQFE 360
VLTAQRLTLL RRAAAGKVGT SELDPFCLAL KNGLQKRAEL REAWRQREHD PLLKKQPANR 420
QPLQCLFDAP SDAPHGAGSE ALPHNVWPLV EEISQIQLQL HETKKVGKPW YQFVLNVYQG 480
CAFQGGEVTC LSWTLFSQRL PMKRRWTLLL RETMNNLGVS DHAVLSRKTP QPY 533
Serial Number (ID): 27
   Length: 539
   Molecular Type: AA
   Feature Location/Qualifier:
      - source, 1..539
         > mol_type, protein
         > organism, Polarella glacialis
Residue: MKRPAATKKA GQAKKKKMDM DRFVREPADA SSESFHEEFR RCLHGLYEQG IFQYDLIQPV 60 
GPGTPVVSTR VTRCLIGEPG ITYKYLGLRM FAHPWKGEKT SESCSAMYQH NQELISKAEE 120
QAVAVGCDAP PYSSCFNLTL INRMDPADSG APNKLGAVSV SWHADSSLVD YSTITVYVAE 180
HDATQDSFGL SPARPCKPCD DPWRVALRHT PNVQGPEKSC PWEVAAQETA KPVKRRAKAK 240
VHTVPIHDGD IYHLIGDFNH HNQHAVLAGR DAVLAVRRGM PVLGGSGTIR YSSTHRVALT 300
EGHSYRSVKQ RCSTALSVAL ADMIPAAAQV HEEQQCSLEL EFEWIRQFFI QGKAHREALP 360
WWHSKMDELV FLWRQFEVLT AQRLTLLRRA AAGKVGTSEL DPFCLALKNG LQKRAELREA 420
WRQREHDPLL KKQPANRQPL QCLFDAPSDA PHGAGSEALP HNVWPLVEEI SQIQLQLHET 480
KKVGKPWYQF VLNVYQGCAF QGGEVTCLSW TLFSQRLPMK RRWKRPAATK KAGQAKKKK 539
Serial Number (ID): 28
   Length: 550
   Molecular Type: AA
   Feature Location/Qualifier:
      - source, 1..550
         > mol_type, protein
         > organism, Guillardia theta
Residue: 
MPKKKRKVQE TLWQRVKEGL EWFDGAIGEG EEEEEAGRIA GNKGGGRRRG FEQALERYYP 60
KFRVIHRDLL PSALEPSTRR ALEVLKDQMG WFRRDYVWVK GKVTQTMVTR LLVGERGCTY 120
KYKGLRLFCR SWETEAAKVP DSACHALAEI RRLNEVLKEK SRKQHECFMA RGEGSGEGAV 180
EGGVNSFNAT LINYFDESIA NHVETRPEPY YGMGRCCISW HADDGVVEQS AIAVYNVIER 240
AEKGEEEELG EVWKVGLKVK WEVDTPGVAV PMVNGDAYVM LSGFNEKNQH CVVCGRGSRF 300
SSTHRVCPQE DMYHNFYCRC RETFLQLHFP WNARGVLQEG EERRACLSKI VASLDALEDL 360
HDQLEFEWLR QFHFQYFDLP PSELDDYWVE VIHVLADLWQ RMEIVVWIWI SCCRRKSFMV 420
GGKELRDRLE RLRQLTSQQK GQGQEVQSSL PFLMKKLLRS EGAGGGREAA GEKAIPEDAL 480
ELVKATLARR LQKRQEHRIW WEENFRKRLS MGRGGEQREG GKRKRKEGRA DGLGFSLLPA 540
NLNSLVSTLK 550
Serial Number (ID): 29
   Length: 548
   Molecular Type: AA
   Feature Location/Qualifier:
      - source, 1..548
         > mol_type, protein
         > organism, Guillardia theta
Residue: 
MQETLWQRVK EGLEWFDGAI GEGEEEEEAG RIAGNKGGGR RRGFEQALER YYPKFRVIHR 60
DLLPSALEPS TRRALEVLKD QMGWFRRDYV WVKGKVTQTM VTRLLVGERG CTYKYKGLRL 120
 
FCRSWETEAA KVPDSACHAL AEIRRLNEVL KEKSRKQHEC FMARGEGSGE GAVEGGVNSF 180
NATLINYFDE SIANHVETRP EPYYGMGRCC ISWHADDGVV EQSAIAVYNV IERAEKGEEE 240
ELGEVWKVGL KVKWEVDTPG VAVPMVNGDA YVMLSGFNEK NQHCVVCGRG SRFSSTHRVC 300
PQEDMYHNFY CRCRETFLQL HFPWNARGVL QEGEERRACL SKIVASLDAL EDLHDQLEFE 360
WLRQFHFQYF DLPPSELDDY WVEVIHVLAD LWQRMEIVVW IWISCCRRKS FMVGGKELRD 420
RLERLRQLTS QQKGQGQEVQ SSLPFLMKKL LRSEGAGGGR EAAGEKAIPE DALELVKATL 480
ARRLQKRQEH RIWWEENFRK RLSMGRGGEQ REGGKRKRKE GRADGLGFSL LPANLNSLVS 540
TLKRKVIK 548
Serial Number (ID): 30
   Length: 551
   Molecular Type: AA
   Feature Location/Qualifier:
      - source, 1..551
         > mol_type, protein
         > organism, Guillardia theta
Residue: 
MRPRKQETLW QRVKEGLEWF DGAIGEGEEE EEAGRIAGNK GGGRRRGFEQ ALERYYPKFR 60
VIHRDLLPSA LEPSTRRALE VLKDQMGWFR RDYVWVKGKV TQTMVTRLLV GERGCTYKYK 120
GLRLFCRSWE TEAAKVPDSA CHALAEIRRL NEVLKEKSRK QHECFMARGE GSGEGAVEGG 180
VNSFNATLIN YFDESIANHV ETRPEPYYGM GRCCISWHAD DGVVEQSAIA VYNVIERAEK 240
GEEEELGEVW KVGLKVKWEV DTPGVAVPMV NGDAYVMLSG FNEKNQHCVV CGRGSRFSST 300
HRVCPQEDMY HNFYCRCRET FLQLHFPWNA RGVLQEGEER RACLSKIVAS LDALEDLHDQ 360
LEFEWLRQFH FQYFDLPPSE LDDYWVEVIH VLADLWQRME IVVWIWISCC RRKSFMVGGK 420
ELRDRLERLR QLTSQQKGQG QEVQSSLPFL MKKLLRSEGA GGGREAAGEK AIPEDALELV 480
KATLARRLQK RQEHRIWWEE NFRKRLSMGR GGEQREGGKR KRKEGRADGL GFSLLPANLN 540
SLVSTLKRPR K 551
Serial Number (ID): 31
   Length: 454
   Molecular Type: AA
   Feature Location/Qualifier:
      - source, 1..454
         > mol_type, protein
         > organism, Ostreococcus tauri
Residue: 
MPKKKRKVTL LLRETMNNLG VSDHAVLSRK TPQPYPDVLG PGDGDAYERV RSFHYDGFVV 60
DDPEILPEAL HDDVARAFDS LLREGEFTHD VLSAGNKVSR TFVTRTLLGD RGTTYHYQKL 120
 
RLFAQPWDGE KRWEYATARR LNEALTRRTR EILRGTRHGE SECEYNVTLI NRMDTREESE 180
VALKDEALFD LGTTSVSWHS DSSLRENSTV AVYSTVDDPE RADWSVALRA LGKQCPALRV 240
PLKDKATYYM CGDFNATHHH AVLTGSSARY SSTHRVGVVE KDTFQYIKRR CLEALALIPD 300
LENKSASLDA KKIQLLAEIH REVEFQWIRM FHLQGDTHAR QHKTYWTQRI AELTEAWDRM 360
EACFRAILSK LKLSPESTPQ PSRAYSMMLY AMKEVKELRD EHVKRISASA YAALPESQRP 420
VDLPQYDDTS PLPFELKPVI YFVEAEQAKL KGES 454
Serial Number (ID): 32
   Length: 441
   Molecular Type: AA
   Feature Location/Qualifier:
      - source, 1..441
         > mol_type, protein
         > organism, Ostreococcus tauri
Residue: 
MPDVLGPGDG DAYERVRSFH YDGFVVDDPE ILPEALHDDV ARAFDSLLRE GEFTHDVLSA 60
GNKVSRTFVT RTLLGDRGTT YHYQKLRLFA QPWDGEKRWE YATARRLNEA LTRRTREILR 120
GTRHGESECE YNVTLINRMD TREESEVALK DEALFDLGTT SVSWHSDSSL RENSTVAVYS 180
TVDDPERADW SVALRALGKQ CPALRVPLKD KATYYMCGDF NATHHHAVLT GSSARYSSTH 240
RVGVVEKDTF QYIKRRCLEA LALIPDLENK SASLDAKKIQ LLAEIHREVE FQWIRMFHLQ 300
GDTHARQHKT YWTQRIAELT EAWDRMEACF RAILSKLKLS PESTPQPSRA YSMMLYAMKE 360
VKELRDEHVK RISASAYAAL PESQRPVDLP QYDDTSPLPF ELKPVIYFVE AEQAKLKGES 420
KRPAATKKAG QAKKKKRKVI K 441
Serial Number (ID): 33
   Length: 490
   Molecular Type: AA
   Feature Location/Qualifier:
      - source, 1..490
         > mol_type, protein
         > organism, Ostreococcus tauri
Residue: 
MKRTPTAEER ERGAKKLRLL EELEDTWLPY LRKVIKPDVL GPGDGDAYER VRSFHYDGFV 60
VDDPEILPEA LHDDVARAFD SLLREGEFTH DVLSAGNKVS RTFVTRTLLG DRGTTYHYQK 120
LRLFAQPWDG EKRWEYATAR RLNEALTRRT REILRGTRHG ESECEYNVTL INRMDTREES 180
EVALKDEALF DLGTTSVSWH SDSSLRENST VAVYSTVDDP ERADWSVALR ALGKQCPALR 240
VPLKDKATYY MCGDFNATHH HAVLTGSSAR YSSTHRVGVV EKDTFQYIKR RCLEALALIP 300
DLENKSASLD AKKIQLLAEI HREVEFQWIR MFHLQGDTHA RQHKTYWTQR IAELTEAWDR 360
 
MEACFRAILS KLKLSPESTP QPSRAYSMML YAMKEVKELR DEHVKRISAS AYAALPESQR 420
PVDLPQYDDT SPLPFELKPV IYFVEAEQAK LKGESRKVIK KRTPTAEERE RGAKKLRLLE 480
ELEDTWLPYL 490
Serial Number (ID): 34
   Length: 791
   Molecular Type: AA
   Feature Location/Qualifier:
      - source, 1..791
         > mol_type, protein
         > organism, Ectocarpus sp
Residue: 
MRPRKTMTTA TTVATTSRLL SPPTSEATTP IQHSNSGMEV STEKTSHKKK RKSKRKRQQQ 60
ERDTSSIPDA SRQSRGSSSS STRPPPPPPP PPPTTTTTTT SKKHSRIKEK PARFVHPHLD 120
AELRRLGRSW LSPGDPLFKP ALDRSYRGVR HEPGGSLPQA VHRGFRQSFD SLHEAGLFLY 180
DTVQAGGKRL SRTFVTRTLV GDAGITYKYL GLRLFAHPWG LGESDFRKGP GDSLADALND 240
PCLADLFSND GSGFDDNDRV ADTLRKVGDL SEWMRLRAGA MLKEEVGEAG GERGSCAFNL 300
TLINRMECSG AKRDLKPDPL FSMGKFSVSW HADSCLQDFS TIGVYHCTDP VPAEPDWGIA 360
LRVSTEARVG GGGGGSGDGD GYGVSNVEKT RRKEAERGVK PAGPKAIASV AASAAGAVPE 420
KQGEQVLTPP LLIPLQSGDA YFLLDDFNHF HEHAVVSGSS TLRYSSTHRV ADTDGNTFSH 480
VQKRCERALA GGGSAAAGGD YHILNLAQVR AEQALLTELE SEWLNQFWVQ GRRHASLHPW 540
WRGPMALVLA TWRELERRTA LTVRRLMSLR GQGGGEGVGP GGGGTAAETA GAEPAAIAKA 600
FETVLRALEV RRESRSRWGA RFADSAYRSL AADLAPVVPA LWPAEGSSGG DVGEEGSPLL 660
LPQSLGGIVS RLRECYTVVY RAASGGKKAG SAKAIAAGAA SNWELFRAAS SSSSSGGGGA 720
GKKHGSALGK QQQQQQQQQQ QQRKMRGLAG QDGLEKTKKR AGGVVKGGGD GGGGRTLKGQ 780
GSRRKDRVRE R 791
Serial Number (ID): 35
   Length: 792
   Molecular Type: AA
   Feature Location/Qualifier:
      - source, 1..792
         > mol_type, protein
         > organism, Ectocarpus sp
Residue: 
MTMTTATTVA TTSRLLSPPT SEATTPIQHS NSGMEVSTEK TSHKKKRKSK RKRQQQERDT 60
SSIPDASRQS RGSSSSSTRP PPPPPPPPPT TTTTTTSKKH SRIKEKPARF VHPHLDAELR 120
RLGRSWLSPG DPLFKPALDR SYRGVRHEPG GSLPQAVHRG FRQSFDSLHE AGLFLYDTVQ 180
 
AGGKRLSRTF VTRTLVGDAG ITYKYLGLRL FAHPWGLGES DFRKGPGDSL ADALNDPCLA 240
DLFSNDGSGF DDNDRVADTL RKVGDLSEWM RLRAGAMLKE EVGEAGGERG SCAFNLTLIN 300
RMECSGAKRD LKPDPLFSMG KFSVSWHADS CLQDFSTIGV YHCTDPVPAE PDWGIALRVS 360
TEARVGGGGG GSGDGDGYGV SNVEKTRRKE AERGVKPAGP KAIASVAASA AGAVPEKQGE 420
QVLTPPLLIP LQSGDAYFLL DDFNHFHEHA VVSGSSTLRY SSTHRVADTD GNTFSHVQKR 480
CERALAGGGS AAAGGDYHIL NLAQVRAEQA LLTELESEWL NQFWVQGRRH ASLHPWWRGP 540
MALVLATWRE LERRTALTVR RLMSLRGQGG GEGVGPGGGG TAAETAGAEP AAIAKAFETV 600
LRALEVRRES RSRWGARFAD SAYRSLAADL APVVPALWPA EGSSGGDVGE EGSPLLLPQS 660
LGGIVSRLRE CYTVVYRAAS GGKKAGSAKA IAAGAASNWE LFRAASSSSS SGGGGAGKKH 720
GSALGKQQQQ QQQQQQQQRK MRGLAGQDGL EKTKKRAGGV VKGGGDGGGG RTLKGQGSRR 780
KDRVRERRKV IK 792
Serial Number (ID): 36
   Length: 841
   Molecular Type: AA
   Feature Location/Qualifier:
      - source, 1..841
         > mol_type, protein
         > organism, Ectocarpus sp
Residue: 
MTLLLRETMN NLGVSDHAVL SRKTPQPYTM TTATTVATTS RLLSPPTSEA TTPIQHSNSG 60
MEVSTEKTSH KKKRKSKRKR QQQERDTSSI PDASRQSRGS SSSSTRPPPP PPPPPPTTTT 120
TTTSKKHSRI KEKPARFVHP HLDAELRRLG RSWLSPGDPL FKPALDRSYR GVRHEPGGSL 180
PQAVHRGFRQ SFDSLHEAGL FLYDTVQAGG KRLSRTFVTR TLVGDAGITY KYLGLRLFAH 240
PWGLGESDFR KGPGDSLADA LNDPCLADLF SNDGSGFDDN DRVADTLRKV GDLSEWMRLR 300
AGAMLKEEVG EAGGERGSCA FNLTLINRME CSGAKRDLKP DPLFSMGKFS VSWHADSCLQ 360
DFSTIGVYHC TDPVPAEPDW GIALRVSTEA RVGGGGGGSG DGDGYGVSNV EKTRRKEAER 420
GVKPAGPKAI ASVAASAAGA VPEKQGEQVL TPPLLIPLQS GDAYFLLDDF NHFHEHAVVS 480
GSSTLRYSST HRVADTDGNT FSHVQKRCER ALAGGGSAAA GGDYHILNLA QVRAEQALLT 540
ELESEWLNQF WVQGRRHASL HPWWRGPMAL VLATWRELER RTALTVRRLM SLRGQGGGEG 600
VGPGGGGTAA ETAGAEPAAI AKAFETVLRA LEVRRESRSR WGARFADSAY RSLAADLAPV 660
VPALWPAEGS SGGDVGEEGS PLLLPQSLGG IVSRLRECYT VVYRAASGGK KAGSAKAIAA 720
GAASNWELFR AASSSSSSGG GGAGKKHGSA LGKQQQQQQQ QQQQQRKMRG LAGQDGLEKT 780
KKRAGGVVKG GGDGGGGRTL KGQGSRRKDR VRERTLLLRE TMNNLGVSDH AVLSRKTPQP 840
Y 841
Serial Number (ID): 37
   Length: 722
   Molecular Type: AA
   Feature Location/Qualifier:
      - source, 1..722
         > mol_type, protein
         > organism, Conferva siliculosa
Residue: 
MPKKKRKVTT TSATTAARSR LPSPPTSAAT TPIQHSNNGT EASPEKTSNK KKAKNKRKRQ 60
QECSIPAASQ QSRGSSSSST RPPPPTTTTL KKHSRIKEKP ARFVHPHLDA ELRRLGRSWL 120
SPGDPLFTPA LDRSYRGVRH EPAGSLPQAV HSGFRQSFDS LHEAGLFLYD TVQAGGKRLS 180
RTFVTRTLVG DAGITYKYLG LRLFAHPWGL GESDFRKGPG DGLADALNDP CLADLFSKDG 240
ASSVRKSKDN LRGSRFDDND RVADTLRKVG DLSEWMRLRA GAMLKEEVRE AGGERGSCAF 300
NLTLINRMEC SGAKRDLKPD PLFSMGKCSV SWHADSCLQD FSTIGVYHCT DPVPAEPDWG 360
IALRVSTEAR VGGDGGGDGD GNGVSNDEKT RRKEAERGLK PAGPKAVASV AASAAEAVPE 420
KQGEQVLTPP LLIPLQSGDA YFLLDDFNHF HEHAVVSGSS TLRYSSTHRV ADTDGNTFSH 480
VQKRCEQALA GGGSAAAGGD DQILNLAQVR AEQALLTELE SEWLNQFWVQ GRRHASLHPW 540
WRGPMALVLA TWRELERRTS LTVQHLMSLR GQDGGEALWP AEGSGGGDVG EEGSTLLLPQ 600
SLGGVISRLR ECYTVVYRAA SGGKKAGSAK AIAAGAASNW ELFRAASSSA SSGGGGAGKK 660
HGSALGKQQQ QQQQRKKRGA AGQDGLEKKK RAGGVVKGGG DGGGGRTSKG QRSRRKGRVR 720
ER 722
Serial Number (ID): 38
   Length: 721
   Molecular Type: AA
   Feature Location/Qualifier:
      - source, 1..721
         > mol_type, protein
         > organism, Conferva siliculosa
Residue: 
MTTTSATTAA RSRLPSPPTS AATTPIQHSN NGTEASPEKT SNKKKAKNKR KRQQECSIPA 60
ASQQSRGSSS SSTRPPPPTT TTLKKHSRIK EKPARFVHPH LDAELRRLGR SWLSPGDPLF 120
TPALDRSYRG VRHEPAGSLP QAVHSGFRQS FDSLHEAGLF LYDTVQAGGK RLSRTFVTRT 180
LVGDAGITYK YLGLRLFAHP WGLGESDFRK GPGDGLADAL NDPCLADLFS KDGASSVRKS 240
KDNLRGSRFD DNDRVADTLR KVGDLSEWMR LRAGAMLKEE VREAGGERGS CAFNLTLINR 300
MECSGAKRDL KPDPLFSMGK CSVSWHADSC LQDFSTIGVY HCTDPVPAEP DWGIALRVST 360
EARVGGDGGG DGDGNGVSND EKTRRKEAER GLKPAGPKAV ASVAASAAEA VPEKQGEQVL 420
TPPLLIPLQS GDAYFLLDDF NHFHEHAVVS GSSTLRYSST HRVADTDGNT FSHVQKRCEQ 480
ALAGGGSAAA GGDDQILNLA QVRAEQALLT ELESEWLNQF WVQGRRHASL HPWWRGPMAL 540
 
VLATWRELER RTSLTVQHLM SLRGQDGGEA LWPAEGSGGG DVGEEGSTLL LPQSLGGVIS 600
RLRECYTVVY RAASGGKKAG SAKAIAAGAA SNWELFRAAS SSASSGGGGA GKKHGSALGK 660
QQQQQQQRKK RGAAGQDGLE KKKRAGGVVK GGGDGGGGRT SKGQRSRRKG RVRERRNKKK 720
K 721
Serial Number (ID): 39
   Length: 727
   Molecular Type: AA
   Feature Location/Qualifier:
      - source, 1..727
         > mol_type, protein
         > organism, Conferva siliculosa
Residue: 
MRNKKKKTTT SATTAARSRL PSPPTSAATT PIQHSNNGTE ASPEKTSNKK KAKNKRKRQQ 60
ECSIPAASQQ SRGSSSSSTR PPPPTTTTLK KHSRIKEKPA RFVHPHLDAE LRRLGRSWLS 120
PGDPLFTPAL DRSYRGVRHE PAGSLPQAVH SGFRQSFDSL HEAGLFLYDT VQAGGKRLSR 180
TFVTRTLVGD AGITYKYLGL RLFAHPWGLG ESDFRKGPGD GLADALNDPC LADLFSKDGA 240
SSVRKSKDNL RGSRFDDNDR VADTLRKVGD LSEWMRLRAG AMLKEEVREA GGERGSCAFN 300
LTLINRMECS GAKRDLKPDP LFSMGKCSVS WHADSCLQDF STIGVYHCTD PVPAEPDWGI 360
ALRVSTEARV GGDGGGDGDG NGVSNDEKTR RKEAERGLKP AGPKAVASVA ASAAEAVPEK 420
QGEQVLTPPL LIPLQSGDAY FLLDDFNHFH EHAVVSGSST LRYSSTHRVA DTDGNTFSHV 480
QKRCEQALAG GGSAAAGGDD QILNLAQVRA EQALLTELES EWLNQFWVQG RRHASLHPWW 540
RGPMALVLAT WRELERRTSL TVQHLMSLRG QDGGEALWPA EGSGGGDVGE EGSTLLLPQS 600
LGGVISRLRE CYTVVYRAAS GGKKAGSAKA IAAGAASNWE LFRAASSSAS SGGGGAGKKH 660
GSALGKQQQQ QQQRKKRGAA GQDGLEKKKR AGGVVKGGGD GGGGRTSKGQ RSRRKGRVRE 720
RRNKKKK 727
Serial Number (ID): 40
   Length: 440
   Molecular Type: AA
   Feature Location/Qualifier:
      - source, 1..440
         > mol_type, protein
         > organism, Fragilariopsis cylindrus
Residue: 
MKRPAATKKA GQAKKKKPKK KRKVLTPEDG VYFQKHLQDH YKGFVHLPVD RLEPRDFHEE 60
FKNSLERLRD AGYYQYDVVM AGGKYSSRTF VKRTLVGNPG ITYKYLGLRL FAHAWSGPGC 120
TPLMKSIGDM NQSMIKMTEQ FPENERCDYN LTLINYMEPT THTKVGFKDE ANYGMGKVSV 180
 
SWHADSSLVD NSSIGVYHCL PTQRNSKWDW KIALRPLAIE SKDNTGSTKR DNKSTTPKPV 240
VVNTKDGDAY FLLGNFNMKH QHCVLAGSQA NRISSTHRVA VTIEDTYDYI LKRVKIARKR 300
FRLQMETIPP SLQSPVVGLD AKVIRYCQRI LTEVEMEWIA QYWLQGDQHD KMRVWWQKPM 360
KTLEAYWCAL EVYTYRLFEF LLSRIASNDT VPIDVVKVLI IEFKTRQSFR EQWDERRSDK 420
IYQRRVSEEF RPVARPIFEN 440
Serial Number (ID): 41
   Length: 451
   Molecular Type: AA
   Feature Location/Qualifier:
      - source, 1..451
         > mol_type, protein
         > organism, Fragilariopsis cylindrus
Residue: 
MLTPEDGVYF QKHLQDHYKG FVHLPVDRLE PRDFHEEFKN SLERLRDAGY YQYDVVMAGG 60
KYSSRTFVKR TLVGNPGITY KYLGLRLFAH AWSGPGCTPL MKSIGDMNQS MIKMTEQFPE 120
NERCDYNLTL INYMEPTTHT KVGFKDEANY GMGKVSVSWH ADSSLVDNSS IGVYHCLPTQ 180
RNSKWDWKIA LRPLAIESKD NTGSTKRDNK STTPKPVVVN TKDGDAYFLL GNFNMKHQHC 240
VLAGSQANRI SSTHRVAVTI EDTYDYILKR VKIARKRFRL QMETIPPSLQ SPVVGLDAKV 300
IRYCQRILTE VEMEWIAQYW LQGDQHDKMR VWWQKPMKTL EAYWCALEVY TYRLFEFLLS 360
RIASNDTVPI DVVKVLIIEF KTRQSFREQW DERRSDKIYQ RRVSEEFRPV ARPIFENKRT 420
PTAEERERGA KKLRLLEELE DTWLPYLRPR K 451
Serial Number (ID): 42
   Length: 461
   Molecular Type: AA
   Feature Location/Qualifier:
      - source, 1..461
         > mol_type, protein
         > organism, Fragilariopsis cylindrus
Residue: 
MRNKKKKKRP AATKKAGQAK KKKLTPEDGV YFQKHLQDHY KGFVHLPVDR LEPRDFHEEF 60
KNSLERLRDA GYYQYDVVMA GGKYSSRTFV KRTLVGNPGI TYKYLGLRLF AHAWSGPGCT 120
PLMKSIGDMN QSMIKMTEQF PENERCDYNL TLINYMEPTT HTKVGFKDEA NYGMGKVSVS 180
WHADSSLVDN SSIGVYHCLP TQRNSKWDWK IALRPLAIES KDNTGSTKRD NKSTTPKPVV 240
VNTKDGDAYF LLGNFNMKHQ HCVLAGSQAN RISSTHRVAV TIEDTYDYIL KRVKIARKRF 300
RLQMETIPPS LQSPVVGLDA KVIRYCQRIL TEVEMEWIAQ YWLQGDQHDK MRVWWQKPMK 360
TLEAYWCALE VYTYRLFEFL LSRIASNDTV PIDVVKVLII EFKTRQSFRE QWDERRSDKI 420
 YQRRVSEEFR PVARPIFENK RPAATKKAGQ AKKKKRNKKK K 461
Serial Number (ID): 43
   Length: 555
   Molecular Type: AA
   Feature Location/Qualifier:
      - source, 1..555
         > mol_type, protein
         > organism, Acanthaster planci
Residue: 
MTLLLRETMN NLGVSDHAVL SRKTPQPYRF LSLNVLRRWM SKMSKRRQTE DLGKEVKKKK 60
LLSEVARGRL QFLMPDHPDF KSLMRKSYFN FAHEPSTGIP VSLHEELTWA LDILRYHGYF 120
YRDWVKVKGQ TVQTPISRTL IGEPGTTYKY LGLRLFTIPW SFGHHKPESE VELASETIGK 180
LNEHYKAVSR RLLRKKTAEQ THLKSSPSQE VAPCSSKDRP RERICVEEPK QMTHGLSIEE 240
GTNFNVVLIN YMDPEDPDLR LKPEPYYGMG PLAVSWHMDG NLVRGSTVAV YNHTCSSDGG 300
DSTTDSDWMV GFKVSWDIET PGVAIPLQTG ESYFMLGDLN DTHQHCVVAG SQARFSTTHR 360
VADCQQGTLS YIQQRCLEAL GNLSQLENGE YELKSFQAGH LDFTETVHNE VEFEWLRQFW 420
MQGSRHAAER GGWVPAMECL EADWNKMEHM TKLAVEEANR LGPEEGSVIA EALLPHLKER 480
RKLRQECLDK FPRRLLETIH RDFLPIHRPK WDNDDPSQPL PFDLKGIIRK MRLLCNGKRI 540
KSPSDSESSD TGLHS 555
Serial Number (ID): 44
   Length: 533
   Molecular Type: AA
   Feature Location/Qualifier:
      - source, 1..533
         > mol_type, protein
         > organism, Acanthaster planci
Residue: 
MRFLSLNVLR RWMSKMSKRR QTEDLGKEVK KKKLLSEVAR GRLQFLMPDH PDFKSLMRKS 60
YFNFAHEPST GIPVSLHEEL TWALDILRYH GYFYRDWVKV KGQTVQTPIS RTLIGEPGTT 120
YKYLGLRLFT IPWSFGHHKP ESEVELASET IGKLNEHYKA VSRRLLRKKT AEQTHLKSSP 180
SQEVAPCSSK DRPRERICVE EPKQMTHGLS IEEGTNFNVV LINYMDPEDP DLRLKPEPYY 240
GMGPLAVSWH MDGNLVRGST VAVYNHTCSS DGGDSTTDSD WMVGFKVSWD IETPGVAIPL 300
QTGESYFMLG DLNDTHQHCV VAGSQARFST THRVADCQQG TLSYIQQRCL EALGNLSQLE 360
NGEYELKSFQ AGHLDFTETV HNEVEFEWLR QFWMQGSRHA AERGGWVPAM ECLEADWNKM 420
EHMTKLAVEE ANRLGPEEGS VIAEALLPHL KERRKLRQEC LDKFPRRLLE TIHRDFLPIH 480
RPKWDNDDPS QPLPFDLKGI IRKMRLLCNG KRIKSPSDSE SSDTGLHSRK VIK 533
Serial Number (ID): 45
   Length: 538
   Molecular Type: AA
   Feature Location/Qualifier:
      - source, 1..538
         > mol_type, protein
         > organism, Acanthaster planci
Residue: 
MRKVIKRFLS LNVLRRWMSK MSKRRQTEDL GKEVKKKKLL SEVARGRLQF LMPDHPDFKS 60
LMRKSYFNFA HEPSTGIPVS LHEELTWALD ILRYHGYFYR DWVKVKGQTV QTPISRTLIG 120
EPGTTYKYLG LRLFTIPWSF GHHKPESEVE LASETIGKLN EHYKAVSRRL LRKKTAEQTH 180
LKSSPSQEVA PCSSKDRPRE RICVEEPKQM THGLSIEEGT NFNVVLINYM DPEDPDLRLK 240
PEPYYGMGPL AVSWHMDGNL VRGSTVAVYN HTCSSDGGDS TTDSDWMVGF KVSWDIETPG 300
VAIPLQTGES YFMLGDLNDT HQHCVVAGSQ ARFSTTHRVA DCQQGTLSYI QQRCLEALGN 360
LSQLENGEYE LKSFQAGHLD FTETVHNEVE FEWLRQFWMQ GSRHAAERGG WVPAMECLEA 420
DWNKMEHMTK LAVEEANRLG PEEGSVIAEA LLPHLKERRK LRQECLDKFP RRLLETIHRD 480
FLPIHRPKWD NDDPSQPLPF DLKGIIRKMR LLCNGKRIKS PSDSESSDTG LHSRKVIK 538
Serial Number (ID): 46
   Length: 489
   Molecular Type: AA
   Feature Location/Qualifier:
      - source, 1..489
         > mol_type, protein
         > organism, Saccoglossus kowalevskii
Residue: 
MRPRKNPRMK RKIPRIPLVT SEGEVAGQLA LPIRKQKLLS AVKETQVKFL SPVDTDYKAL 60
VAENYDNFVV QPGTELSSEL HTRLQQGFET LLTNGYLYQH RIRFRNKTVM TPVSRTLIGE 120
PGMTYRYLNL RLFALPWTFD DKKKLSDVEK ACNAFYELNT YFKDHVNKLI TEKFASSTGR 180
KIDETDDSLK TIIQRNTEFK ATILNYMDPS EIRLKQEPYY NMGDIAVSWH KDESLVDGST 240
VAVYNHVPME ARQDCSKWKV GLKVAWDITT PAVCTPLNNK DCYYMLDTMN DTHQHCVIAG 300
TLPRYSSTHR VVESSDSTLC YIQQRCQTAL DNIGYKDQIP YLKSLETDVL QLIEEIHSEI 360
EFEWLRQFWM QGTQHSQERQ YWLKPMEQLQ EDWKTMELMT KLMIDIVVKK KDQSHMIAKI 420
LLPSLVERNE LRNEWMNRYS EKEFYHIPEE HRPIYEPCWT DANSEMPLPF DLSQHIDIIQ 480
ECMDTNDSQ 489
Serial Number (ID): 47
   Length: 491
   Molecular Type: AA
   Feature Location/Qualifier:
      - source, 1..491
         > mol_type, protein
         > organism, Saccoglossus kowalevskii
Residue: 
MNPRMKRKIP RIPLVTSEGE VAGQLALPIR KQKLLSAVKE TQVKFLSPVD TDYKALVAEN 60
YDNFVVQPGT ELSSELHTRL QQGFETLLTN GYLYQHRIRF RNKTVMTPVS RTLIGEPGMT 120
YRYLNLRLFA LPWTFDDKKK LSDVEKACNA FYELNTYFKD HVNKLITEKF ASSTGRKIDE 180
TDDSLKTIIQ RNTEFKATIL NYMDPSEIRL KQEPYYNMGD IAVSWHKDES LVDGSTVAVY 240
NHVPMEARQD CSKWKVGLKV AWDITTPAVC TPLNNKDCYY MLDTMNDTHQ HCVIAGTLPR 300
YSSTHRVVES SDSTLCYIQQ RCQTALDNIG YKDQIPYLKS LETDVLQLIE EIHSEIEFEW 360
LRQFWMQGTQ HSQERQYWLK PMEQLQEDWK TMELMTKLMI DIVVKKKDQS HMIAKILLPS 420
LVERNELRNE WMNRYSEKEF YHIPEEHRPI YEPCWTDANS EMPLPFDLSQ HIDIIQECMD 480
TNDSQRNKKK K 491
Serial Number (ID): 48
   Length: 545
   Molecular Type: AA
   Feature Location/Qualifier:
      - source, 1..545
         > mol_type, protein
         > organism, Saccoglossus kowalevskii
Residue: 
MKRTPTAEER ERGAKKLRLL EELEDTWLPY LNPRMKRKIP RIPLVTSEGE VAGQLALPIR 60
KQKLLSAVKE TQVKFLSPVD TDYKALVAEN YDNFVVQPGT ELSSELHTRL QQGFETLLTN 120
GYLYQHRIRF RNKTVMTPVS RTLIGEPGMT YRYLNLRLFA LPWTFDDKKK LSDVEKACNA 180
FYELNTYFKD HVNKLITEKF ASSTGRKIDE TDDSLKTIIQ RNTEFKATIL NYMDPSEIRL 240
KQEPYYNMGD IAVSWHKDES LVDGSTVAVY NHVPMEARQD CSKWKVGLKV AWDITTPAVC 300
TPLNNKDCYY MLDTMNDTHQ HCVIAGTLPR YSSTHRVVES SDSTLCYIQQ RCQTALDNIG 360
YKDQIPYLKS LETDVLQLIE EIHSEIEFEW LRQFWMQGTQ HSQERQYWLK PMEQLQEDWK 420
TMELMTKLMI DIVVKKKDQS HMIAKILLPS LVERNELRNE WMNRYSEKEF YHIPEEHRPI 480
YEPCWTDANS EMPLPFDLSQ HIDIIQECMD TNDSQKRTPT AEERERGAKK LRLLEELEDT 540
WLPYL 545
END

## Claims

1. The modified RNA demethylase is **characterized by** the addition of at least one peptide sequence at the N-terminus and/or the C-terminus, as compared to the original RNA demethylase derived from algae or invertebrates, thereby obtaining the modified RNA demethylase

2. The modified RNA demethylase as described in claim 1, wherein the peptide sequence has any of the following amino acid sequence characteristics:
a) contains at least three amino acids selected from the group consisting of H, K, and R;
b) contains PY and at least one amino acid selected from the group consisting of H, K, and R.

3. The modified RNA demethylase as described in claim 1, **characterized in that** when the peptide sequence has the amino acid sequence feature of a), then at least two amino acids selected from H, K, or R are consecutive, or contain V or I.

4. The modified RNA demethylase as described in claim 1, **characterized in that** the peptide sequence has any of the following amino acid sequence features: (K/R)(K/R)X₁₋₂(K/R), (K/R)X₁₋₂(K/R)(K/R), (K/R)(K/R)X₁₀₋₁₂(K/R)₂₋₅, (R/K/H)X₂₋₅PY, or K(V/I)XKX₁₋₂(K/H/R), wherein X is any amino acid.

5. The modified RNA demethylase as described in claim 1, **characterized in that** the peptide sequence includes at least one of RPRK, RKVIK, RNKKKK, PKKKRKV, KRPAATKKAGQAKKKK, TLLLRETMNNLGVSDHAVLSRKTPQPY, KRTPTAEERERGAKKLRLLEELEDTWLPYL.

6. The modified RNA demethylase as described in claim 1, **characterized in that** the original RNA demethylase includes at least one of its homologs or variants/conjugates with equivalent function.

7. The modified RNA demethylase as described in claim 1, **characterized in that** the original RNA demethylase promotes the demethylation of N6-methyladenine in RNA.

8. The modified RNA demethylase as described in claim 1, **characterized in that** the modified RNA demethylase is as shown in the sequence list Seq.ID.No:13 ~Seq.ID.No:48.

9. The modified RNA demethylase as described in claim 1, **characterized in that** the invertebrate includes at least one of Acanthaster planci or sacoglossan.

10. The modified RNA demethylase as described in claim 1, **characterized in that** the algae include at least one of blue algae, red algae, cryptophytes, dinoflagellates, chiysophytes, yellow-green algae, diatoms, brown algae, euglenoids, green algae, and charophytes.

11. Nucleic acid encoding the modified RNA demethylase as described in claim 1.

12. The nucleic acid as described in claim 11, **characterized in that** the nucleic acid is DNA.

13. The use of the modified RNA demethylase described in claim 1 or the use of nucleic acid described in claim 11for introduction into plants.

14. The use as described in claim 13, **characterized in that** the use includes any one of yield increase or biomass increase.

15. The use as described in claim 13, **characterized in that** the plants include at least one of rapeseed plant, tomato, lettuce, beet, rice, com, soybean plant, potato, wheat, millet, sugarcane, sorghum, cassava, tobacco, alfalfa, rubber grass, cotton, flax, sunflower, camelina, nutsedge, cannabis, and poplar.

16. A plant cultivation method, **characterized by** introducing the modified RNA demethylase as described in claim 1 or the nucleic acid as described in claim 11 into the initial plant, thereby obtaining a target plant with optimized traits compared to the initial plant.

17. The plant cultivation method as described in claim 16, **characterized in that** the trait optimization includes at least one of yield increase and biomass increase.

18. The plant cultivation method as described in claim 16, **characterized in that** the modified RNA demethylase according to claim 1 or the nucleic acid is introduced in an amount excessive for the initial plant.

19. The plant cultivation method as described in claim 16, **characterized in that** the initial plant is selected from at least one of rapeseed plant, tomato, lettuce, beet, rice, corn, soybean plant, potato, wheat, millet, sugarcane, sorghum, cassava, tobacco, alfalfa, rubber grass, cotton, flax, sunflower, camelina, nutsedge, cannabis, and poplar.

20. The plant cultivation method as described in claim 16, **characterized in that** the plant part subjected to introduction treatment includes at least one of plant organs, plant tissues, or plant cells.

21. The plant cultivation method as described in claim 16, **characterized in that** the plant part subjected to introduction treatment is selected from plant meristem.

22. The plant cultivation method as described in claim16, **characterized in that** the plant part subjected to introduction treatment includes at least one of the plant's nucleus and cytoplasm.

23. The plant cultivation method as described in claim 16, **characterized in that** the introduction treatment method includes introducing into the plant a vector carring the modified RNA demethylase as described in claim 1 or the nucleic acid as described in claim 11.

24. The plant cultivation method as described in claim 16, **characterized in that** the trait optimization degree is Y compared to the initial plant, where Y is the multiple of increase compared to the initial plant at the same period, Y≥3.50, preferably Y≥3.80, more preferably Y≥4.00.

25. A product derived from the target plant obtained by the plant cultivation method as described in Claim 16 and subjected to inactivation treatment.
